# EUROPEAN PATENT SPECIFICATION

(11) **EP 1 289 567 B1**
(45) Date of publication and mention of the grant of the patent: **30.07.2008**
(21) Application number: 01939720.7
(22) Date of filing: 31.05.2001
(51) Int. Cl.: A61K 48/00, G01N 21/00

(54) **LYOPHILIZABLE AND ENHANCED COMPACTED NUCLEIC ACIDS**
LYOPHILISIERBARE UND VERBESSERTE KOMPAKTIERTE NUKLEINSÄURE
ACIDES NUCLEIQUES COMPACTES AMELIORES LYOPHILISABLES

(30) Priority: 31.05.2000 US 207949 P; 01.05.2001 US 287419 P
(43) Date of publication of application: 12.03.2003
(73) Proprietor: Copernicus Therapeutics, Inc., Cleveland OH 44106-3052 (US)
(72) Inventor: COOPER, Mark, J., Moreland Hills, OH 44022 (US); KOWALCZYK, Thomasz, H., University Heights, OH 44118 (US); PASUMARTHY, Murali, K., Twinsburg, OH 44087 (US); COSTELLO, Maureen, Beachwood, OH 44122-7567 (US)
(74) Representative: Tombling, Adrian George
(86) International application number: PCT/US2001/017499
(87) International publication number: WO 2001/092580

(56) References cited:
- EP-A- 1 031 626
- WO-A-97/30731
- WO-A-98/46274
- KICHLER A ET AL: "INFLUENCE OF THE DNA COMPLEXATION MEDIUM ON THE TRANSFECTION EFFICIENCY OF LIPOSPERMINE/DNA PARTICLES" GENE THERAPY, MACMILLAN PRESS LTD., BASINGSTOKE, GB, vol. 5, 1998, pages 855-860, XP001030902 ISSN: 0969-7128
- DATABASE SCISEARCH [Online] AN - 2000:868868, NOEL T ET AL: "High compacted DNA - polymer complexes via new polynorbornene polycationic latexes with acetate counterion" retrieved from STN Database accession no. 868868 XP002185734 & LANGMUIR, (14 NOV 2000) VOL. 16, NO. 23, PP. 8980-8983. PUBLISHER: AMER CHEMICAL SOC, 1155 16TH ST, NW, WASHINGTON, DC 20036. ISSN: 0743-7463., UNIV TOULOUSE 3, UMR CNRS 5623, LAB IMRCP, 118 ROUTE NARBONNE, F-31062 TOULOUSE, FRANCE (Reprint);UNIV TOULOUSE 3, UMR CNRS 5623, LAB IMRCP, F-31062 TOULOUSE, FRANCE

## Description

### BACKGROUND OF THE INVENTION

Despite the promise of preclinical models for systemic gene therapy to liver, lung, and other tissues, there is currently no commercial gene therapy product on the market. The failure of most human gene therapy clinical trials to treat metabolic disorders and cancer has been ascribed to the relative inefficiency of viral and non-viral gene transfer systems. Viral vectors have been used for most gene therapy studies because of their ability to efficiently infect cells in tissue culture. However, an enormous payload of particles needs to be applied in an intravenous injection to transduce cells in vivo, and toxicities of viral vectors are well documented [I], including a recent lethal toxicity that occurred following a portal vein injection of recombinant adenovirus [2]. In contrast, non-viral systems are generally felt to be safe although inefficient. There is a growing consensus that non-viral systems will be the vector of choice for in vivo applications once gene transfer efficiency is improved.

Several barriers restrict non-viral methods of gene transfer, including: i) particle stability in blood and interstitial tissues; ii) ability of the gene transfer particle to exit capillaries and travel to parenchymal cells; iii) cell entry via receptor-mediated endocytosis or cell fusion; iv) stability in and escape from endosomal and lysosomal compartments; v) diffusion rate in the cytoplasm; vi) nuclear pore transit; and vii) "uncoating" of DNA to permit biological function in the nucleus. For example, numerous publications have documented the failure of non-viral methods to transfect post-mitotic, growth-arrested cells [3-11], presumably because the intact nuclear membrane of non-dividing cells restricts entry of naked DNA into the nucleus via the 25 nm nuclear pore [12-13].

Thus there is a continuing need in the art for improved formulations for delivery of genes to animals and humans. In addition, there is a need in the art for formulations which will be stable to storage and retain biological activity.

### SUMMARY OF THE INVENTION

These and other objects of the invention are provided by one or more of the embodiments disclosed below. In one embodiment of the invention a method of estimating the colloidal stability of a preparation of compacted nucleic acids is provided. A turbidity parameter of a solution of compacted nucleic acid is determined. The turbidity parameter is defined as the slope of a straight line obtained by plotting log of apparent absorbance of light versus log of incident wavelength of the light. The wavelength used is between about 330 nm and 420 nm. A preparation is identified as colloidally stable if a turbidity parameter of less than -3 is determined. A preparation is identified as colloidally unstable if a turbidity parameter of greater than or equal to -3 is determined.

According to another aspect of the invention a non-naturally occurring composition comprising unaggregated nucleic acid complexes is provided. Each complex consists essentially of a single nucleic acid molecule and one or more polycation molecules. The polycation molecules have a counterion selected from the group consisting of acetate, bicarbonate, and chloride. The complex is compacted to a diameter which is less than (a) double the theoretical diameter of a complex of said single nucleic acid molecule and a sufficient number of polycation molecules to provide a charge ratio of about 1:1, in the form of a condensed sphere, or (b) 30 nm, whichever is larger. Optionally, the one or more polycation molecules of the unaggregated nucleic acid complexes are CK15-60P10, wherein acetate is used as a counterion. CK15-60P10 is a polyamino acid polymer of one N-terminal cysteine and 15-60 lysine residues, with a molecule of polyethylene glycol having an average molecular weight of 10 kDa attached to the cysteine residue.

According to another aspect of the invention a method of preparing a composition comprising unaggregated nucleic acid complexes is provided. Each complex consists essentially of a single nucleic acid molecule and one or more polycation molecules. The polycation molecules have a counterion selected from the group consisting of acetate, bicarbonate, and chloride. The complex is compacted to a diameter which is less than (a) double the theoretical diameter of a complex of said single nucleic acid molecule and a sufficient number of polycation molecules to provide a charge ratio of about 1:1, in the form of a condensed sphere, or (b) 30 nm, whichever is larger. The nucleic acid is mixed with the polycation having acetate, bicarbonate, or chloride as a counterion, at a salt concentration sufficient for compaction of the complex. Optionally, the one or more polycation molecules of the unaggregated nucleic acid complexes are CK15-60P10, wherein acetate is used as a counterion. CK15-60P10 is a polyamino acid polymer of one N-terminal cysteine and 15-60 lysine residues, with a molecule of polyethylene glycol having an average molecular weight of 10 kDa attached to the cysteine residue.

An additional embodiment of the invention is provided as a method of preparing a composition comprising unaggregated nucleic acid complexes. Each complex consists essentially of a single nucleic acid molecule and one or more polycation molecules having an acetate, bicarbonate or chloride counterion. A nucleic acid molecule is mixed with a polycation molecule at a salt concentration sufficient for compaction of the complex to a diameter which is less than double the theoretical minimum diameter of a complex of said single nucleic acid molecule and a sufficient number of polycation molecules to provide a charge ratio of about 1:1, in the form of a condensed sphere, or 30 nm, whichever is larger. Unaggregated nucleic acid complexes are formed. Optionally, the one or more polycation molecules of the unaggregated nucleic acid complexes are CK15-60P10, wherein acetate is used as a counterion. CK15-60P10 is a polyamino acid polymer of one N-terminal cysteine and 15-60 lysine residues, with a molecule of polyethylene glycol having an average molecular weight of 10 kDa attached to the cysteine residue.

Also provided by the present invention is a non-naturally occurring composition comprising unaggregated nucleic acid complexes. Each complex consists essentially of a single nucleic acid molecule and one or more polycation molecules. The polycation molecules have a counterion selected from the group consisting of acetate, bicarbonate, and chloride. The nucleic acid molecule encodes at least one functional protein. Said complex is compacted to a diameter which is less than double the theoretical minimum diameter of a complex of said single nucleic acid molecule and a sufficient number of polycation molecules to provide a charge ratio of about 1:1, in the form of a condensed sphere, or 30 nm, whichever is larger. Optionally, the one or more polycation molecules of the unaggregated nucleic acid complexes are CK15-60P10, wherein acetate is used as the counterion. CK15-60P10 is a polyamino acid polymer of one N-terminal cysteine and 15-60 lysine residues, with a molecule of polyethylene glycol having an average molecular weight of 10 kDa attached to the cysteine residue.

Another non-naturally occurring composition comprising unaggregated nucleic acid complexes is also provided. Each complex consists essentially of a single double-stranded cDNA molecule and one or more polycation molecules. Said polycation molecules have a counterion selected from the group consisting of acetate, bicarbonate, and chloride. The cDNA molecule encodes at least one functional protein. The complex is compacted to a diameter which is less than double the theoretical minimum diameter of a complex of said single cDNA molecule and a sufficient number of polycation molecules to provide a charge ratio of about 1:1, in the form of a condensed sphere, or 30 nm, whichever is larger. The nucleic acid complexes are optionally associated with a lipid. Optionally, the one or more polycation molecules of the unaggregated nucleic acid complexes are CK15-60P10, wherein acetate is used as the counterion. CK15-60P10 is a polyamino acid polymer of one N-terminal cysteine and 15-60 lysine residues, with a molecule of polyethylene glycol having an average molecular weight of 10 kdal attached to the cysteine residue.

Another non-naturally occurring composition comprising unaggregated nucleic acid complexes is provided by the present invention. Each complex consists essentially of a single nucleic acid molecule and one or more polycation molecules. The polycation molecules have a counterion selected from the group consisting of acetate, bicarbonate, and chloride. The nucleic acid molecule encodes at least one antisense nucleic acid. The complex is compacted to a diameter which is less than double the theoretical minimum diameter of a complex of said single nucleic acid molecule and a sufficient number of polycation molecules to provide a charge ratio of about 1:1, in the form of a condensed sphere, or 30 nm, whichever is larger. Optionally, the one or more polycation molecules of the unaggregated nucleic acid complexes are CK15-60P 10, wherein acetate is used as the counterion. CK15-60P10 is a polyamino acid polymer of one N-terminal cysteine and 15-60 lysine residues, with a molecule of polyethylene glycol having an average molecular weight of 10 kDa attached to the cysteine residue.

According to another aspect of the invention a non-naturally occurring composition comprising unaggregated nucleic acid complexes is provided. Each complex consists essentially of a single nucleic acid molecule and one or more polycation molecules. The polycation molecule has a counterion selected from the group consisting of acetate, bicarbonate, and chloride. The nucleic acid molecule is an RNA molecule. The complex is compacted to a diameter which is less than double the theoretical minimum diameter of a complex of said single nucleic acid molecule and a sufficient number of polycation molecules to provide a charge ratio of about 1:1, in the form of a condensed sphere, or 30 nm, whichever is larger. Optionally, the one or more polycation molecules of the unaggregated nucleic acid complexes are CK15-60P10, wherein acetate is used as the counterion. CK15-60P10 is a polyamino acid polymer of one N-terminal cysteine and 15-60 lysine residues with a molecule of polyethylene glycol having an average molecular weight of 10 kDa is attached to the cysteine residue.

Another aspect of the invention provided here is a method of preparing a composition comprising unaggregated nucleic acid complexes. Each complex consists essentially of a single nucleic acid molecule and one or more polycation molecules. A nucleic acid molecule is mixed with a polycation molecule in a solvent to form a complex. The mixing is performed in the absence of added salt, whereby the nucleic acid forms soluble complexes with the polycation molecule without forming aggregates. Each complex consists essentially of a single nucleic acid molecule and one or more polycation molecules. The complexes have a diameter which is less than double the theoretical minimum diameter of a complex of the single nucleic acid molecule and a sufficient number of polycation molecules to provide a charge ratio of about 1:1, in the form of a condensed sphere, or 30 nm, whichever is larger. The polycation has acetate, bicarbonate, or chloride as a counterion. Optionally, the one or more polycation molecules of the unaggregated nucleic acid complexes are CK15-60P10, wherein acetate is used as the counterion. CK15-60P10 is a polyamino acid polymer of one N-terminal cysteine and 15-60 lysine residues with a molecule of polyethylene glycol having an average molecular weight of 10 kDa is attached to the cysteine residue.

Finally, the present invention provides the use of the compositions of the invention in the manufacture of a medicament for preventing or treating a disease or other clinical condition in a subject. A prophylactically or therapeutically effective amount of a composition is admimistered intramuscularly or to the lung. The composition comprises: unaggregated nucleic acid complexes, each complex consisting essentially of a single nucleic acid molecule and one or more polycation molecules, said polycation molecule having acetate, chloride, or bicarbonate as a counterion, wherein said complex is compacted to a diameter which is less than (a) double the theoretical minimum diameter of a complex of said single nucleic acid molecule and a sufficient number of polycation molecules to provide a charge ratio of about 1:1, in the form of a condensed sphere, or (b) 30 nm, whichever is larger. The nucleic acid is one whose integration, hybridization or expression within target cells of the subject prevents or treats the disease or other clinical condition. Optionally, the one or more polycation molecules of the unaggregated nucleic acid complexes are CK15-60P10, wherein acetate is used as the counterion. CK15-60P10 is a polyamino acid polymer of one N-terminal cysteine and 15-60 lysine residues with a molecule of polyethylene glycol having an average molecular weight of 10 kDa is attached to the cysteine residue.

The present invention thus provides the art with improved analytical and therapeutic techniques for delivery of DNA to cells by providing compacted nucleic acid compositions having improved stability and transfectability properties.

### BRIEF DESCRIPTION OF THE DRAWINGS

Fig. 1 shows intramuscular (IM) injection results using TFA (trifluoroacetate) and acetate as counterions for polylysine used to compact DNA.
Fig. 2 shows intramuscular injection results using TFA (trifluoroacetate) as counterion for polylysine used to compact DNA.
Fig. 3 shows intramuscular injection results using acetate as counterions for polylysine used to compact DNA.
Fig. 4 shows intramuscular injection results using acetate as counterions for polylysine used to compact DNA.
Fig. 5 shows a variety of parameters varying and their effectiveness in IM injections, including size of polylysine (CK), polyethylene glycol substitution.
Fig. 6 shows intra-tracheal instillation of 100 ug naked and 100 ug compacted DNA compared as to amount of expression in the lung of the instilled gene (luciferase) as a function of time after gene transfer.
Fig. 7A shows intra-tracheal instillation of naked and compacted DNA compared as to amount of expression in the lung of the instilled gene (luciferase) as a function of time after gene transfer. Fig. 7B shows plot of data above background from Fig. 7A.
Fig. 8 shows turbidity parameter plots as a function of size of polylysine used in compaction and counterion
Fig. 9A, Fig. 9B, and Fig. 9C show a comparison of serum stability, turbidity parameter, and sedimentation, for various formulations of compacted nucleic acids. Fig. 9D tabulates the results.
Fig. 10 shows the influence of counterion on the morphology of PEG-substituted CK30 compacted DNA as shown under the electron microscope.
Fig. 11 shows the stability of PLAS*min*^{™} DNA upon freezing and lyophilization. Particles were tested with sucrose, trehalose, or no excipient. Particles were tested with and without polyethylene glycol, and with TFA or acetate as the counterion. DNA stability was assessed by a low (3400 x g x 1 min) spin to pellet aggregates, and monitoring the absorbance of DNA in the supernatant. Stability with acetate as the counterion surpassed other formulations in the absence of excipient.
Fig. 12 shows assessment of the turbidity parameter before and after lyophilization using various excipients, counterions, and with or without polyethylene glycol. Sucrose and trehalose are very effective in maintaining the properties of the pre-lyophilization particles. PEG-acetate similarly was effective in maintaining the properties.
Fig. 13 shows a visualization of particles under the electron microscope. For particles made with CK30-PEG10k acetate in the presence of 0.5 M trehalose, the rod-like compacted particles look identical before and after lyophilization and rehydration.
Fig. 14 shows a visualization of particles under the electron microscope. For particles made with CK30 TFA in the presence of 0.5M sucrose, the ellipsoidal particles of compacted DNA look identical before and after lyophilization and rehydration.
Fig. 15 shows the results of gene transfer experiments using lyophilized PLAS*min*™ complexes. Luciferase enzyme was encoded by the complexes and its activity was measured as a means of monitoring gene transfer. While sucrose and trehalose were effective in protecting the gene transfer activity to all particles, particles which contained polyethylene glycol (10 kDa) and acetate as a counterion were surprisingly stable to lyophilization, even in the absence of cryoprotectant excipient (disaccharide).
Fig. 16 shows a comparison of the colloidal stability of CK30P10K and CK45P10K DNA complexes compacted using various counterions in 0.9% NaCl. Colloidal stability is evaluated by sedimentation and turbidity parameter.
Fig. 17 shows an electron micrograph of plasmid DNA compacted by CK45P10 with chloride as a counterion. Magnification 40,000. The bar shows 100 nm.
Fig. 18 shows an agarose gel electrophoresis of DNA compacted by PEG-ylated polylysine (CK30P10K) with various counterions. The influence of counterions on the effective net charge of the condensed DNA can be seen by the migration of the compacted DNA through the gel. Fig. 18 also shows the serum stability of the CK30P10K-DNA complexes with each of the different counterions.
Fig. 19 shows in vivo expression of luciferase plasmid compacted by various counterion forms of PEG-ylated polylysine (CK30P10K) after intramuscular application. Each point represents one animal. The solid line indicates background signal of luciferase assay. Dose was 100 µg DNA.
Fig. 20 shows in vivo expression of luciferase plasmid compacted by various forms of PEG-ylated polylysine after intranasal application. Acetate, bicarbonate, and TFA forms of CD30P10K and chloride form of CK45P10K were used. The acetate formulation was prepared either in saline or water. Each point represents one animal. The solid line indicates background signal of luciferase assay. Dose was 100 µg DNA.

### DETAILED DESCRIPTION OF THE INVENTION

U.S. Patents 5,844,107 5,877,302, 6,008,336, 6,077,835, 5,972,901, 6,200,801, and 5,972,900 and applications Serial Nos. 60/145,970, 09/722,340, 09/311,553 and 60/207,949 form the state of the art. US 5 844 107 and US 5 877 302 disclose complexes of nucleic acid and polycation wherein each complex comprises a single nucleic acid molecule.

Counterions of polycations used to compact nucleic acids profoundly affect shape of particles formed. Shape is associated with differential serum nuclease resistance and colloidal stability. A surrogate for determining such properties which is easy to measure is the turbidity parameter. Moreover, shape affects the suitability and efficacy of compacted nucleic acid complexes for transfecting cells by various routes into a mammalian body.

The counterion used in making compacted nucleic acid complexes also has a significant effect on the stability of the complexes to lyophilization. Since lyophilization is a common process to render biologicals readily transportable and storage stable, this finding has significant ramifications. Typically, polyamino acid polymers contain trifluoroaceate (TFA) as a counterion. However, this counterion is far less beneficial than acetate for purposes of lyophilization of nucleic acid polymers, as shown below. Particles made using acetate retain their unaggregated nature, *i.e.,* stay in solution better, after lyophilization and rehydration, retain their shape, and retain their gene transfer potential.

Particles according to the present invention contain nucleic acids, preferably a single nucleic acid molecule. The nucleic acid may be DNA or RNA, may be double or single-stranded, may be protein coding or anti-sense coding or non-coding. Nucleic acids also include analogs of RNA and DNA which are modified to enhance the resistance to degradation *in vivo.* A preferred analogue is a methylphosphonate analogue of the naturally occurring mononucleosides. More generally, the mononucleoside analogue is any analogue whose use results in oligonucleotides which have the advantages of (a) an improved ability to diffuse through cell membranes and/or (b) resistance to nuclease digestion within the body of a subject (Miller, P. S. et al., Biochemistry 20:1874-1880 (1981)). Such nucleoside analogues are well-known in the art. The nucleic acid molecule may be an analogue of DNA or RNA. The present invention is not limited to use of any particular DNA or RNA analogue, provided it is capable of fulfilling its therapeutic purpose, has adequate resistance to nucleases, and adequate bioavailability and cell take-up. DNA or RNA may be made more resistant to in vivo degradation by enzymes, e.g., nucleases, by modifying internucleoside linkages (e.g., methylphosphonates or phosphorothioates) or by incorporating modified nucleosides (e.g., 2'-0-methylribose or 1'-alpha- anomers). The methods used for forming the particles are as disclosed in U.S. Patents 5,844,107, 5,877,302, 6,008,336, 6,077,835, 5,972,901, 6,200,801, and 5,972,900 and applications Serial Nos. 60/145,970, 09/722,340, 09/311553 and 60/207949.

Polycations according to the present invention preferably comprise polyamino acids such as polylysine and derivatives of polylysine. The polycation may contain from 15-60 lysine residues, preferably in the ranges of 15-30, 30-45, or 45-60 residues. Preferred derivatives ofpolylysine are CK15, CK30, CK45, which have an additional cysteine residue attached to polylysine polymers of length 15, 30, and 45 residues, respectively. Other amino acids can be readily attached to polylysine without departing from the spirit of the invention. Other polycationic amino acid polymers can be used such as polyarginine, or copolymers of arginine and lysine. Polymers of non-protein amino acids, such as ornithine or citrulline, could also be used. Any pharmaceutically approved or appropriate polycation can be used including but not limited to protamine, histones, polycationic lipids, putrescine, spermidine, spermine, peptides, and polypeptides. The polycation may also contain a targeting moiety, which is typically a ligand which binds to a receptor on a particular type of cell. The targeting ligand may be a polyamino acid or other chemical moiety. Specificity of interaction of the ligand and the receptor is important for purposes of targeting.

Conditions for making compacted nucleic acid particles are disclosed in the aforementioned patents and applications. The conditions may include from 0-1 M salt. The preferred salt is NaCl. Other chaotropic salts can be used as long as they are tolerated by the animal (or cells) to which they will be administered. Suitable agents include Sodium sulfate (Na.sub.2 SO.sub.4), Lithium sulfate (Li.sub.2 SO.sub.4), Ammonium sulfate ((NH.sub.4).sub.2 SO.sub.4, Potassium sulfate (K.sub.2 SO.sub.4), Magnesium sulfate (MgSO.sub.4), Potassium phosphate (KH.sub.2 PO.sub.4), Sodium phosphate (NaH.sub.2 PO.sub.4), Ammonium phosphate (NH.sub.4 H.sub.2 PO.sub.4), Magnesium phosphate (MgHPO.sub.4), Magnesium chloride (Mg Cl.sub.2), Lithium chloride (LiCI), Sodium chloride (NaCl), Potassium chloride (KCI), Cesium chloride (CaCI), Ammonium acetate, Potassium acetate, Sodium acetate, Sodium fluoride (NaF), Potassium fluoride (KF), Tetramethyl ammonium chloride (TMA-Cl), Tetrabutylammonium chloride (TBA-Cl), Triethylammoniym chloride (TEA-Cl), and Methyltriethylammonium chloride (MTEA-Cl).

If a Target Cell Binding Moiety (TBM) is used, it must bind specifically to an accessible structure (the "receptor") of the intended target cells. It is not necessary that it be absolutely specific for those cells, however, it must be sufficiently specific for the conjugate to be therapeutically effective. Preferably, its cross-reactivity with other cells is less than 10%, more preferably less than 5%.

There is no absolute minimum affinity which the TBM must have for an accessible structure of the target cell, however, the higher the affinity, the better. Preferably, the affinity is at least 10.sup.3 liters/mole, more preferably, at least 10.sup.6 liters/mole.

The TBM may be an antibody (or a specifically binding fragment of an antibody, such as an Fab, Fab, V.sub.M, V.sub.L or CDR) which binds specifically to an epitope on the surface of the target cell. Methods for raising antibodies against cells, cell membranes, or isolated cell surface antigens are known in the art: (a). production of immune spleen cells: immunization with soluble antigens Hurrell, J. G. R. (1982) Monoclonal Antibodies: Techniques and Applications. CRC Press, Boca Raton, Fla. (b). immunization with complex antigens: membranes, whole cells and microorganisms. Hurrell, J. G. R. (1982) Monoclonal Antibodies: Techniques and Applications. CRC Press, Boca Raton, Fla. (c). production of monoclonal supernatants and ascites fluids. Andrew, S. M. and Titus, J. A. (1991). Purification of Immunoglobulin G. in Current Protocols in Immunology (J. E. Coligan, A. M. Kruisbeek, D. H. J. Margulies, E. M. Shevach and W. Strober, ed.) pp. A.3.9-A.3.12. Greene Publishing Wiley-Interscience, New York. (d). production of polyclonal antiserum in rabbit. Garvey J. S., Cremer, N. E. and Sussdorf, D. H (eds) (1977) Methods in Immunology: A Laboratory Text for Instruction and Research, Third Edition. W. A. Benjamin, North Hampton, Mass. (e). production of anti-peptide antibodies by chemical coupling of synthetic peptides to carrier proteins Jemmerson, R., Morrow, P. I., Klinman, N. I and Patterson, Y. (1985). Analysis of an evolutionary conserved site on mammalian cytochrome C using synthetic peptides. Proc. Natl Acad. Sci, U.S.A. 82, 1508-1512.

The TBM may be a lectin, for which there is a cognate carbohydrate structure on the cell surface. The target binding moiety may be a ligand which is specifically bound by a receptor carried by the target cells. One class of ligands of interest are carbohydrates, especially mono- and oligosaccharides. Suitable ligands include galactose, lactose and mannose. Another class of ligands of interest are peptides (which here includes proteins), such as insulin, epidermal growth factor(s), tumor necrosis factor, prolactin, chorionic gonadotropin, FSH, LH, glucagon, lactoferrin, transferrin, apolipoprotein E, gp120 and albumin. The following table lists preferred target binding moieties for various classes of target cells:

| Target Cells | Target Binding Moiety |
|---|---|
| liver cells | galactose |
| Kupffer cells | mannose |
| macrophages | mannose |
| lung | Fab fragment vs. polymeric immunoglobulin receptor (Pig R) |
| adipose tissue | insulin |
| lymphocytes | Fab fragment vs. CD4 or gp120 |
| enterocyte | Vitamin B12 |
| muscle | insulin |
| fibroblasts | mannose-6-phosphate |
| nerve cells | Apolipoprotein E |

Use of a target binding moiety is not strictly necessary in the case of direct injection of compacted nucleic acid complex. The target cell in this case is passively accessible to the compacted complex by the injection of the complex to the vicinity of the target cell. Target binding moieties can be attached to lysine residues, cysteine residues, or PEG using covalent or non-covalent interactions.

It has been found that the counterion provided in association with the polycation profoundly affects shape, and that shape is associated with physiologically important properties for delivery of nucleic acids. For example, trifluoroacetate (TFA) particles form spheroids and short rods of less than about 50 nm. Acetate leads to longer rods of 100 to 200 nm. Chloride leads to particles which are longer and skinnier than acetate particles. Bicarbonate leads to a mixture of rods of 100-200 nm and toroids. Bromine is typically supplied with reagent grade polylysine. It is believed that bromine is inferior to other cations as described herein, especially with respect to physiological acceptability. Counterions can be supplied to or substituted on polycations by means of chromatography or dialysis, for example. For example, the polycation can be bound to an ion exchange resin and eluted with the desired counterion. Any method known in the art can be used for this purposed. Interestingly, it has been found that once a particle has been compacted into a particular shaped particle, removal and replacement of the counterion, such as by dialysis, does not significantly alter the shape once assumed. Thus a favorable shape can be obtained with a particle using a non-optimum counterion for physiological purposes and the counterion can be replaced with a superior counterion, while retaining the shape obtained during compaction with the original counterion. The favorable affects on nucleic acids of the counterions may not require compaction. Thus the polycations and counterions can be used with non-compacted nucleic acids as well.

The behavior of these different shaped particles in gene delivery in animals varies significantly. Acetate particles are superior, for example, to TFA particles for delivery to muscle and lung. Delivery to other locations in the body may also be accomplished. These include, without limitation, administrations which are intratracheal, by inhalation, intradermal, topical, by eyedrops, subcutaneous, intrathecal, by enema, enteral, intravenous, intraarterial, intralymphatic, intraperitoneal, intrapleural, intravesicular, intraarticular, intracardiac, intracranial, intratumor, direct to an organ, by eardrops, by nosedrops, intraurethral, endoscopically to the upper gastrointestinal tract, to the sigmoid, or to the colon, by cystoscopy, by thorascope, by arthroscope, by mediastinoscopy, by endoscopic retrograde chlolangiopancreatography, by Omaya reservoir, by angiography including cardiac catheterization and cerebral angiography, intrauterine, intravaginal, to the bone marrow, to hair follicles, to the vitreous and aqueous humor, to the sinuses, to the ureter/pelvis of the kidney, to the fallopian tube, and to lymph nodes.

The complexes have a diameter which is less than double the theoretical minimum diameter of a complex of the single nucleic acid molecule and a sufficient number of polycation molecules to provide a charge ratio of about 1:1, in the form of a condensed sphere. For the purposes of this invention, "about 1:1" encompasses from 1.5:1 to 1:1.5.

Turbidity parameter can be assessed by determining the absorbance of a composition. In a preferred embodiment a Zeiss MCS501 UV-Vis spectrometer is used. Other spectrometers as are known in the art can be substituted. Suitable wavelengths for collection absorbance measurements are between about 330nm and 420 nm.

The invention is explained in particular applications in the examples which follow.

### EXAMPLES

### Example 1

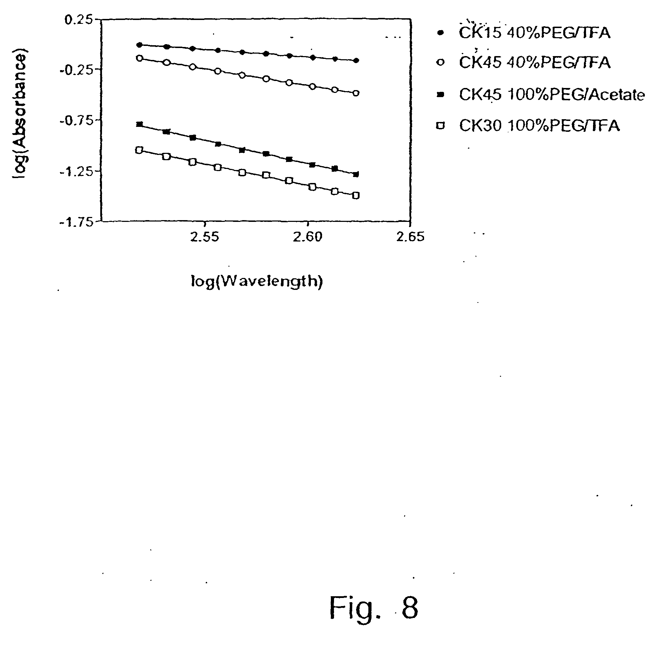

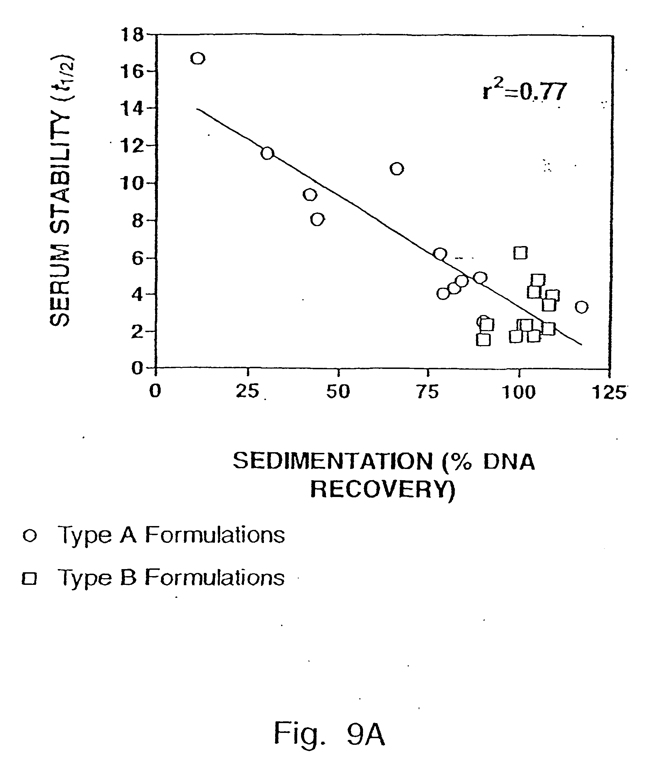

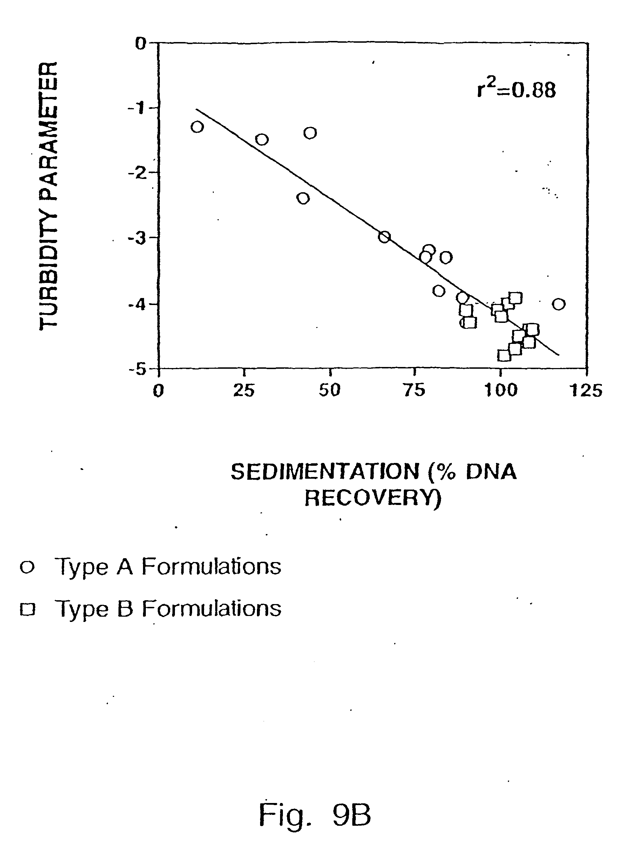

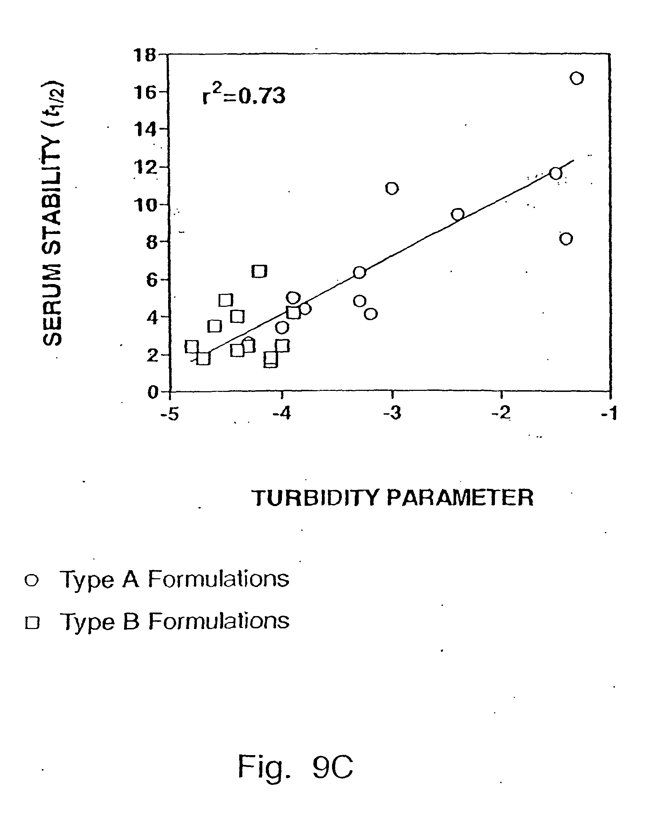

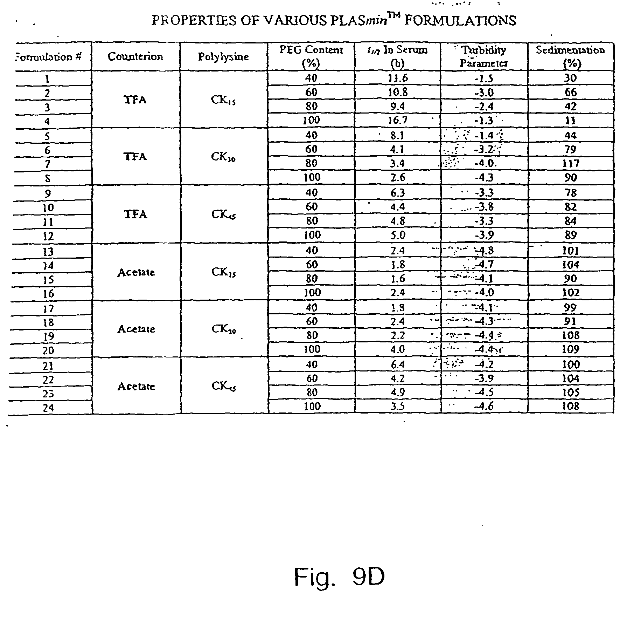

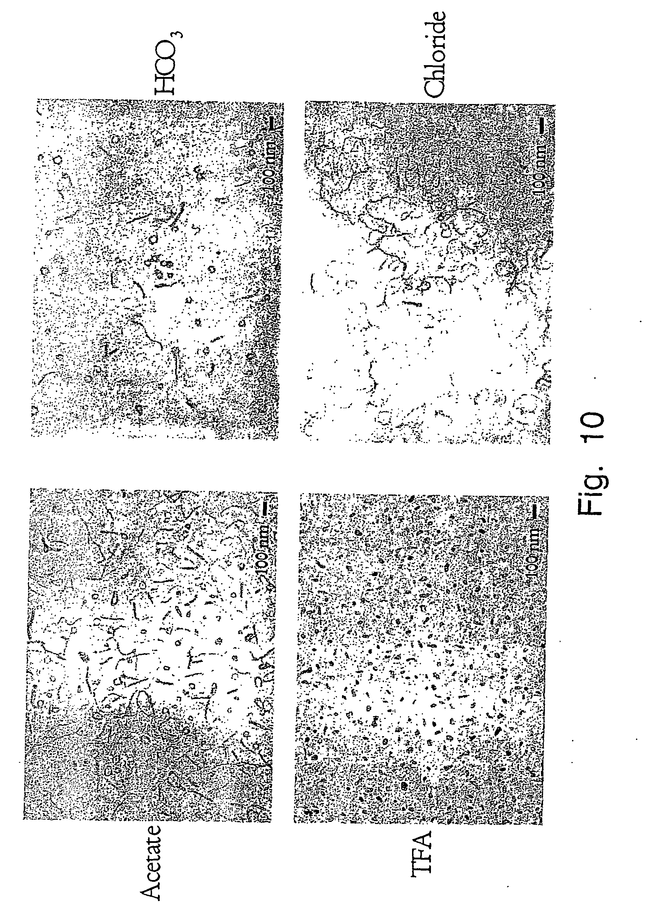

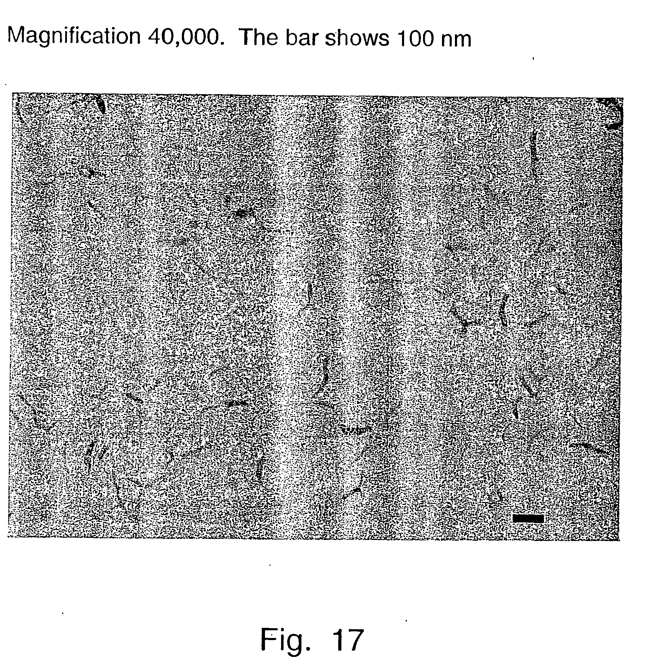

Resistance to serum nucleases is, among other properties, an important feature of any effective gene therapy vector designed to be administered systemically. Ideally, engineering this resistance should not compromise other desirable properties of a vector, such as its small size and colloidal stability. We have developed reagents and methods that permit us to reproducibly compact plasmid DNA with polylysine-polyethylene glycol (PEG) conjugates to form small particles having defined morphology (PLAS*min*^{™} complexes). Some of these formulations are stable in serum and do not aggregate in physiologic saline. By changing components and conditions of the compaction procedure, size and shape of the particles can be modified. To evaluate potential correlations between serum stability and the physical state of PLAS*min*^{™} complexes, we have prepared a matrix of 24 formulations using polylysines of various lengths and substituted with PEG to various extents. Fig. 9D. Polylysines having exactly 15, 30, and 45 residues were obtained by solid-phase synthesis. These polymers contained an N-terminal cysteine residue that was used to conjugate PEG. Various mixtures of PEG-substituted and non-substituted polylysines we re used to obtain different PLAS*min*^{™} complexes. Stability of the complexes in 75% mouse serum was tested by incubating compacted DNA at 37 °C for up to 5 days and determining half-life of DNA degradation. Simultaneously, physical characteristics of the complexes in 150 mM NaCl were determined. Morphology was visualized by transmission electron microscopy (Fig. 10 and Fig. 17). DNA condensed with acetate and bicarbonate salts of CK30 polylysine assumed forms of long (100-300 nm) and narrow (10-20 nm) rods and relaxed toroids (~50-100 nm diameter, 10-20 nm width); the TFA salt resulted in much shorter rods (<60 nm by 20-30 nm) and small globules (20-30 nm); the chloride form of CK30 did not compact DNA at all (Fig. 10), while CK45/chloride (Fig. 17) gave results similar to CK30/acetate. Colloidal instability (tendency to aggregate) was evaluated by a sedimentation assay. Additionally, light scattering of solutions containing PLAS*min*^{™} complexes was measured and expressed as a turbidity parameter (Fig. 8). We found that all PLAS*min*^{™} complexes (Fig. 9A) were much more stable in serum than naked DNA. The half-life for compacted DNA ranged from ~2-17 hr, while naked DNA was completely digested within a few minutes. We also found a correlation (r²=0.77) between half-life of degradation and colloidal instability of PLAS*min*^{™} complexes: particles that tended to aggregate were more resistant to nucleases. The tendency to aggregate also correlated with morphology of the complexes: rod-like complexes did not aggregate; thus, they all showed very similar serum stability, independent of their composition (t_{1/2}~2-5 hr). In contrast, spherical complexes showed various extents of tendency to aggregate depending on polylysine chain-length and PEG content. There was little difference in serum stability between small globules and rod-like particles. In agreement with the prediction that aggregated particles should scatter various light wavelengths differently than small complexes, we found a good correlation (r²=0.88) between colloidal instability of PLAS*min*^{™} complexes and turbidity of their solutions (Fig. 9B): stable complexes had turbidity parameter around -4 to -5 (in accordance with the Rayleigh law), while for the largest and least stable particles this value increased to -1.3. Consequently, the turbidity parameter also correlated with the half-life of DNA degradation in serum (r²=0.73; Fig. 9C). Thus, we conclude that the turbidity parameter, which is easy to determine, can be conveniently used to preliminarily screen various formulations of compacted DNA and predict their colloidal stability as well as serum stability.

### Example 2 (for information only)

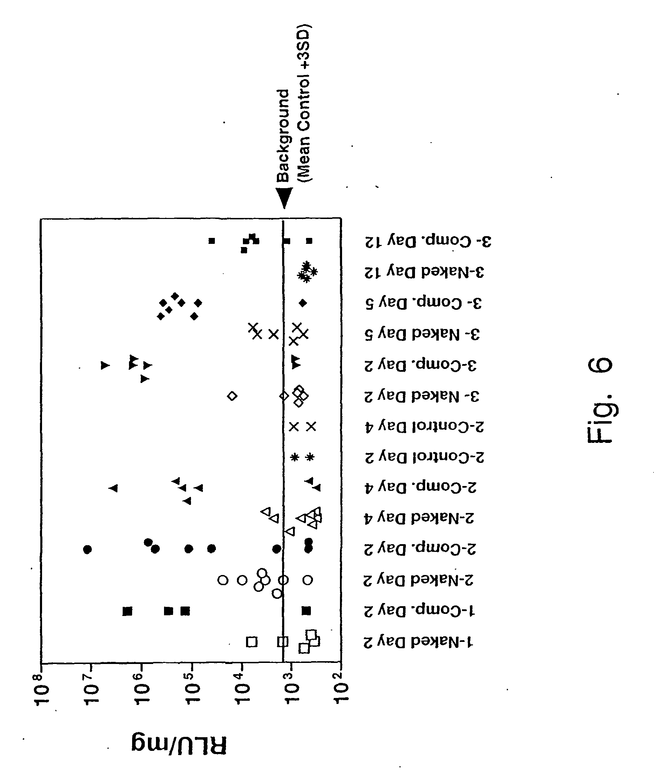

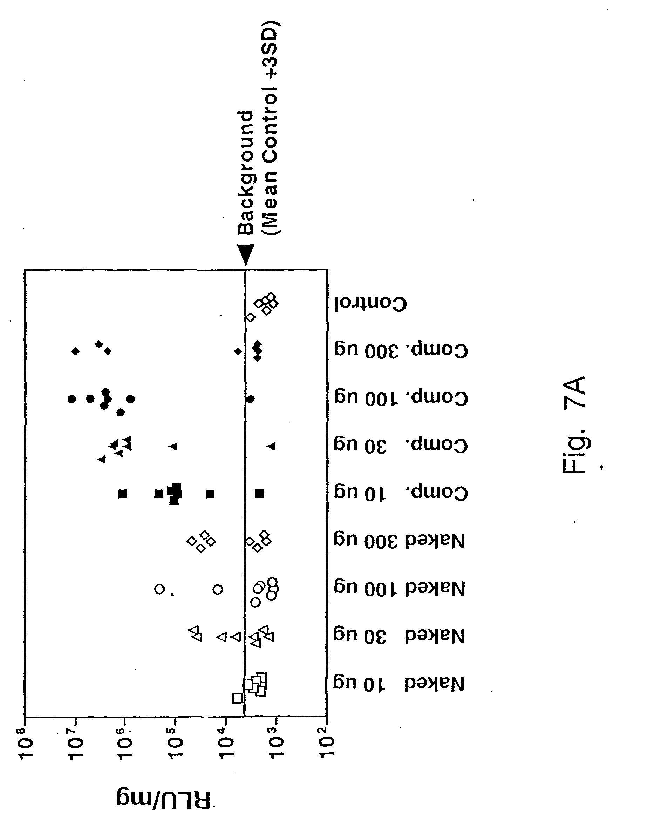

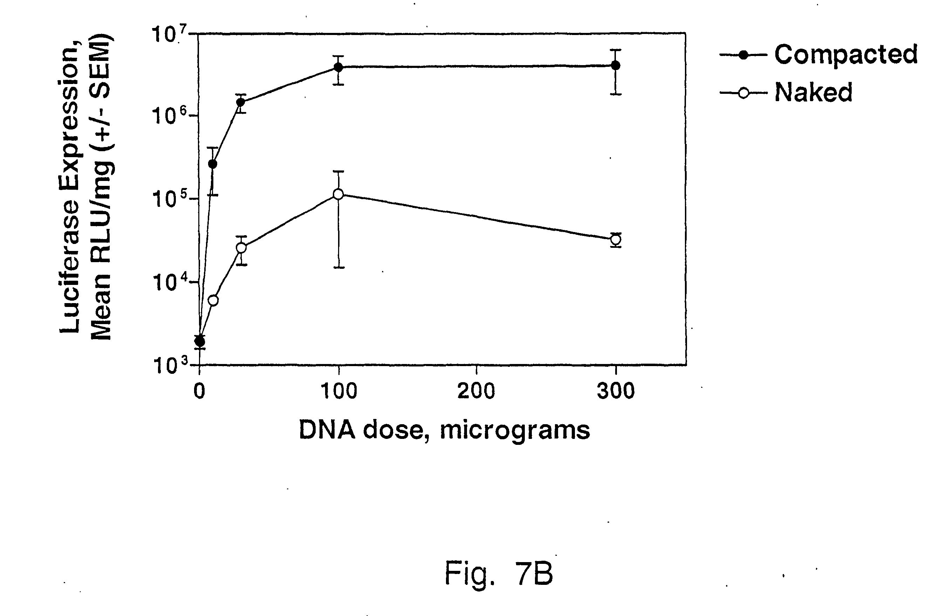

Effective gene transfer to lung would facilitate therapies for pulmonary diseases, such as cystic fibrosis, and may provide a potent means for administering mucosal vaccines. Although direct instillation of naked DNA into mouse airways generates measurable transgene expression, the level of expression is low, and the duration of expression is short. We have developed reagents and formulation methods that compact single molecules of plasmid DNA into 20-25 nm particles (PLAS*min*^{™} complexes). Unlike naked DNA, these complexes are protected from nuclease digestion and are stable in serum. Additionally, PLAS*min*^{™} complexes do not aggregate in physiologic saline and can be concentrated to over 12 mg/ml of DNA. To determine if PLAS*min*^{™} complexes would generate significant levels of gene expression in lung, we instilled naked and PLAS*min*^{™} complexes into the lungs of C57BL/6J mice via direct intratracheal administration. These compacted particles consisted of plasmid DNA and PEG-substituted polylysine polymers consisting of 30 lysine residues. The plasmid construct encoded a luciferase reporter gene transcriptionally controlled by a CMV enhancer, an elongation factor 1-alpha (EF1-alpha) promoter, EF1-alpha intron 1, the RU5 translational enhancer from HTLV I, and an SV40 late polyadenylation signal. A DNA dose of 100 ug was administered in 25 or 50 ul of 150 mM NaCl. At 2, 4, 5, or 12 days following gene transfer, extracts were prepared from both lungs and luciferase activity was measured as relative light units per mg of protein (Fig. 6). Whereas naked DNA generated a signal of approximately 4,000 RLU/mg on day 2 and 1,100 RLU/mg on day 4, PLAS*min*^{™} complexes generated approximately 1,100,000 RLU/mg on day 2, and 630,000 rlu/mg on day 4. Gene expression persisted for at least 12 days after gene transfer, although at lower levels. These compacted DNA particles produced 400-fold enhanced gene expression compared to naked DNA on day 2, and over 1,300-fold improved gene expression on day 4. In contrast to whole lung extracts, less gene expression was noted in trachea, and no expression in liver (data not shown). In dose response studies, peak levels of transgene expression was observed using a 100 ug dose (Fig. 7). In summary, we have determined that PLAS*min*^{™} complexes effectively deliver and express transgenes in mouse lung following direct intra-tracheal administration. In studies in progress, the beta-galactosidase reporter gene is being utilized to define the cell type(s) being transfected. PLAS*min*^{™} complexes may provide an appropriate gene transfer method for diverse pulmonary diseases and/or mucosal vaccines.

### Example 3

Gene transfer in muscle cells following an intramuscular injection provides a means of safe and effective vaccination, and provides therapeutic levels of recombinant proteins, such as factor IX, factor VIII, or alpha-1 anti-trypsin.

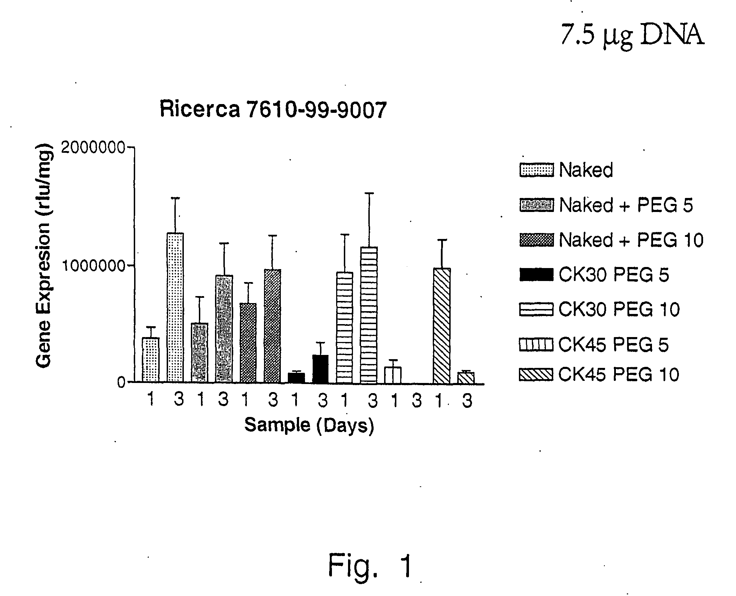

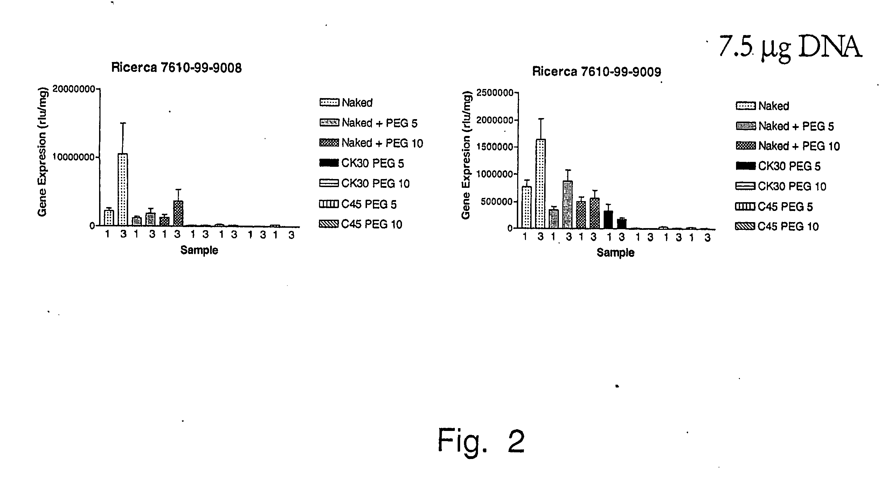

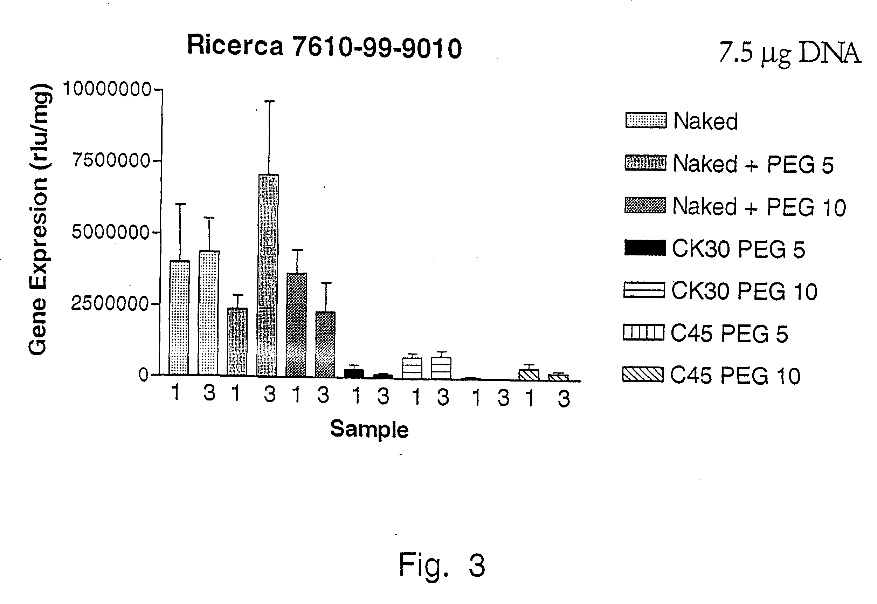

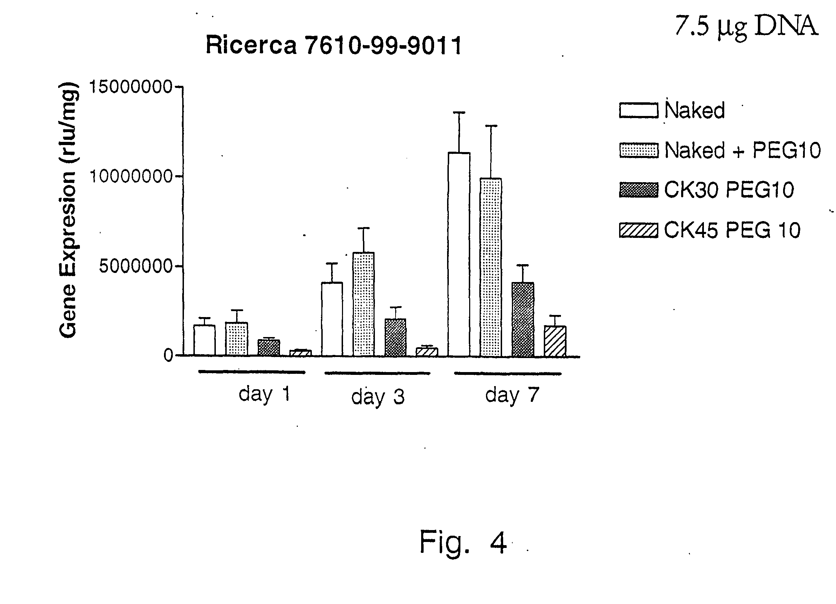

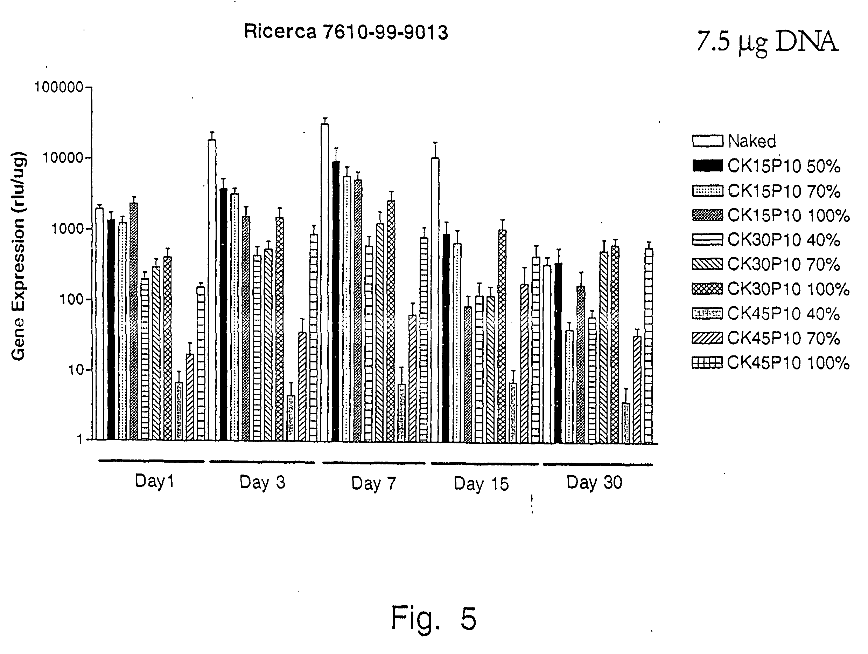

To optimize formulations of PLAS*min*^{™} DNA for intramuscular administration, various preparation of compacted DNA encoding the luciferase reporter gene were administered to CD2 mice by single injection in the tibialis anterior muscle. Gene expression was assayed at various days post gene transfer and is presented as relative light units (RLU)/mg protein. In Figure 1, expression of compacted DNA formulated with the acetate salt of CK30 polycation (complexed with PEG 10 kD) was enhanced, as measured by luciferase activity on both days 1 and 3, compared to other preparations of DNA formulated with the TFA salt of CK30 or CK45. To define further the roles of counterion type, length of polylysine, and percent substitution of polyethylene glycol (PEG), additional experiments were conducted. Animals received IM injections of TFA complexes consisting of either CK30 or CK45, and PEG sizes of either 5 or 10 kD. Figure 2) Luciferase activity was significantly less than that observed for CK30, PEG 10 kD, acetate complexes in Figure 1. The enhanced gene expression of complexes prepared using the acetate salt of CK30, PEG 10 kD, was confirmed. (Figure 3) In this experiment, the CK30 polycation generated better luciferase activity than the CK45 polymer, and CK30 yielded higher levels of luciferase activity when complexed with 10 kD rather than 5 kD PEG. The duration of gene expression produced by acetate complexes consisting of either CK30 or CK45, both complexes with PEG 10 kD, were next evaluated, and the results are shown in Figure 4. In this study, the CK30 polycation gave the best level of reporter gene activity, and the level of activity was better on day 7 than days 1 or 3. A variety of acetate complexes were tested for gene activity as shown in Figure 5. These formulations included CK15, CK30, and CK45 polycations complexed with various percentages of PEG 10 kD. A time course to 30 days was performed. Although gene expression on days 1, 3, and 7 appeared better using CK15 compared to CK30, the particle sizes of some CK15 complexes were larger than 30 nm or two times the theoretical diameter of a complex of said single nucleic acid molecule and a sufficient number of polycation molecules to provide a charge ratio of about 1:1, in the form of a condensed sphere. For days 1, 3, 7, and 15, at least one preparation of CK30 compacted DNA was superior to any CK45 preparation. For CK30, the 100% PEG 10 kD complexes generated better reporter gene activity than either the 70% or 40% substitutions. In summary, the best formulation of compacted DNA in these studies was the acetate salt of CK30 polycation having a 100% substitution with PEG 10 kD.

### Example 4

Prior to injection, animals are anesthetized by intraperitoneal injection with a rodent cocktail of ketamine, xylazine, and acepromazine. A volume of 150 ul anesthetic is administered per mouse, at a concentration of 21.5 mg/ml ketamine, 10.7 mg/ml xylazine, and 0.36 mg/ml acepromazine. The final dose is 0.32 mg ketamine, 1.6 mg xylazine, and 0.054 mg acepromazaine per mouse.

A volume of 25 ml of each plasmid DNA formulation is administered intratracheally to each animal using a 22-gauge needle. A plastic catheter is placed in the trachea of the mice via a percutaneous approach. The resulting does per animal is 300 ug, 100 ug, 30 ug, and 10 ug DNA per mouse.

After injection, animals are anesthetized by carbon dioxide and sacrificed. The animals are bled and rinsed intra-arterially with phosphate buffered saline. The lungs, trachea, and liver are isolated and rinsed in the saline. Tissue samples are immediately frozen on liquid nitrogen, and then stored at -70 °C.

Lung tissue is homogenized using Polytron in lysis buffer. Protein concentration is determined. Luciferase activity of the homogenates is determined by luciferase assay.

### Example 5

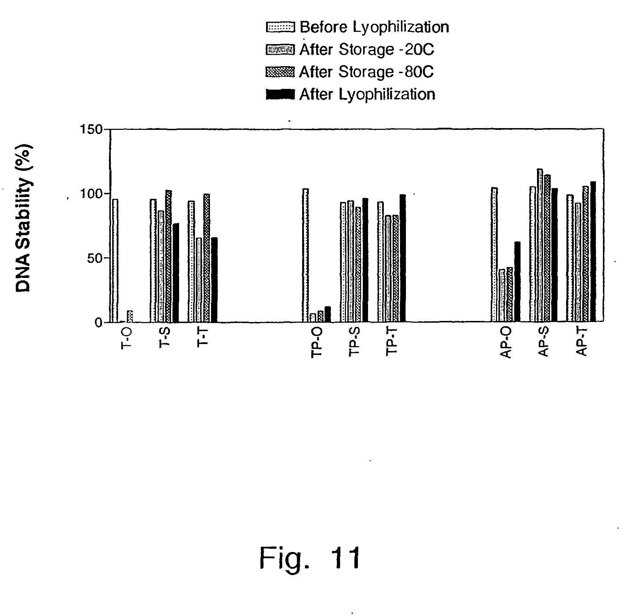

The stability of PLAS*min*^{™}DNA upon freezing and lyophilization was assessed. Particles were tested with sucrose, trehalose, or no excipient. Particles were tested with and without polyethylene glycol, and with TFA or acetate as the counterion to the polyethylene glycol. DNA stability was assessed by a low (3400 x g x 1 min) spin to pellet aggregates, and monitoring the absorbance of DNA in the supernatant. See Fig. 11. Stability of the complexes with acetate as the counterion surpassed other formulations in the absence of excipient.

### Example 6

The turbidity parameter is defined as the slope of a straight line obtained by plotting log of apparent absorbance of light versus log of incident wavelength of the light. The wavelength used is between about 330 nm and 420 nm. A preparation is identified as colloidally stable if a turbidity parameter of less than -3 is determined. A preparation is identified as colloidally unstable if a turbidity parameter of greater than or equal to -3 is determined.

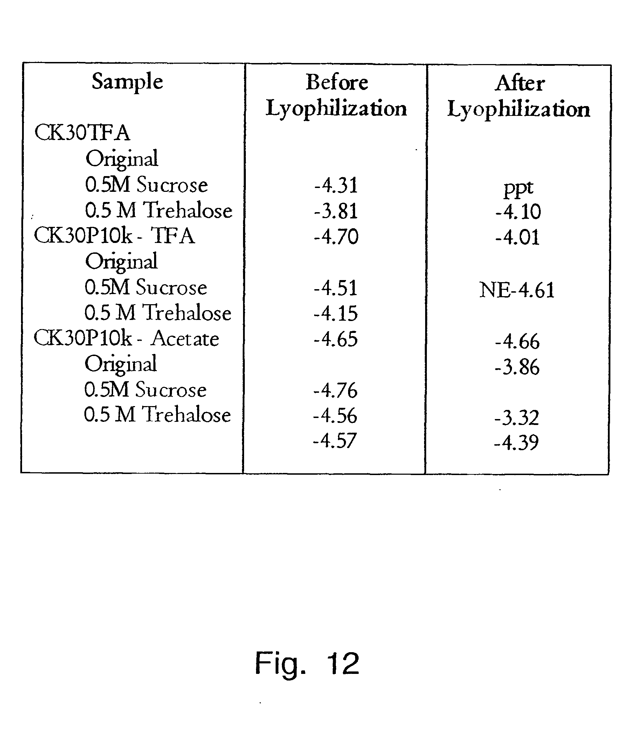

The turbidity parameter of the compacted nucleic acid particles was assessed before and after lyophilization using various excipients, counterions, and with or without polyethylene glycol. See Fig. 12. Sucrose and trehalose were found to be very effective in maintaining the properties of the pre-lyophilization particles. PEG-acetate similarly was effective in maintaining these properties.

### Example 7

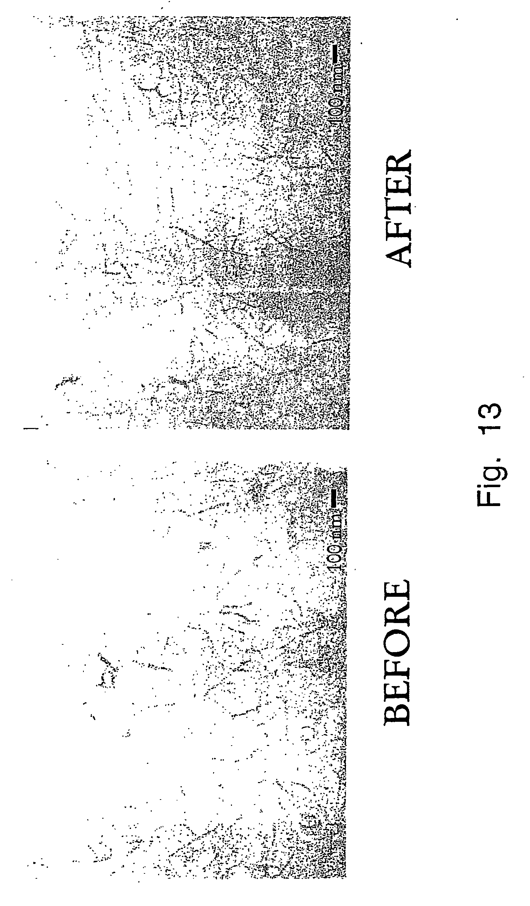

Particles were observed under the electron microscope before and after lyophilization. See Fig. 13. Particles made with CK30-PEG10k acetate in the presence of 0.5 M trehalose look similarly rod-like before and after lyophilization and rehydration.

### Example 8 (for information only)

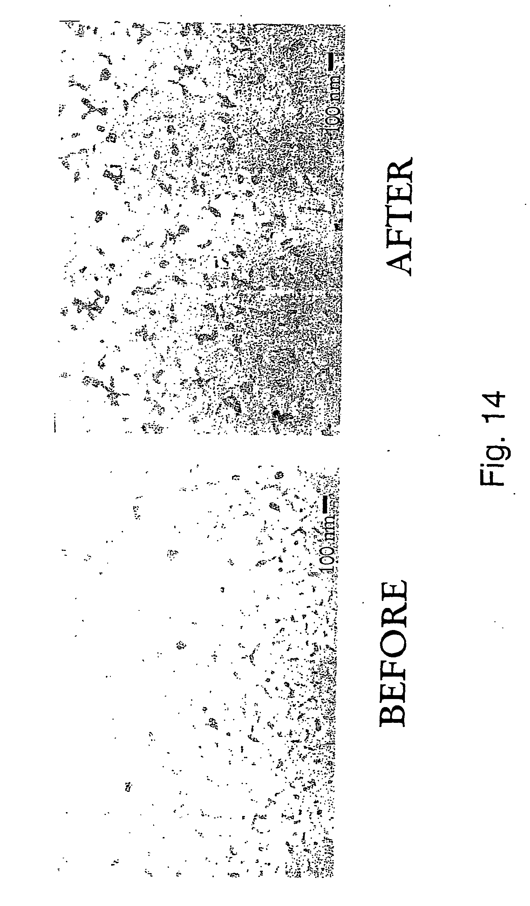

Particles were observed before and after lyophilization and rehydration under the electron microscope. The ellipsoidal particles of compacted DNA made with CK30 TFA (counterion) in the presence of 0.5M sucrose look identical before and after lyophilization and rehydration. See Fig. 14.

### Example 9

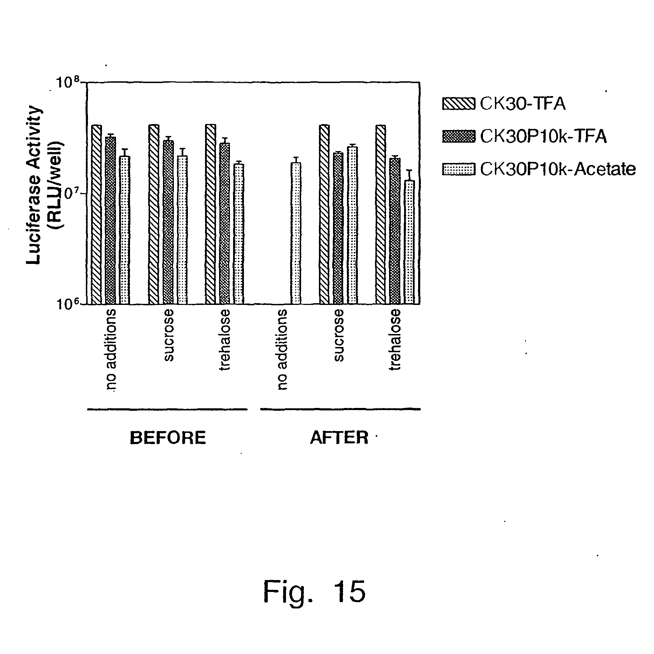

Gene transfer experiments using lyophilized and rehydrated PLAS*min*™ complexes were performed, comparing them to pre-lyophilization preparations. Luciferase enzyme was encoded by the complexes and its activity was measured as a means of monitoring gene transfer. While sucrose and trehalose were effective in protecting the gene transfer activity to all particles, particles which contained polyethylene glycol (10 kDa) and acetate as a counterion were surprisingly stable to lyophilization, even in the absence of cryoprotectant excipient (disaccharide). See Fig. 15.

### Example 10

Polylysines having an N-terminal cysteine and exactly 30 or 45 lysine residues (CK30 or CK45, respectively) were obtained as trifluoroacetate (TFA) salts by solid-phase synthesis. The cysteine residue was then used to conjugate polyethylene glycol (MW 10,000) to form PEG-ylated polylysines CK30P10K and CK45P10K. The TFA counterion was exchanged with acetate, bicarbonate, or chloride by gel filtration. DNA was condensed by these polylysines, dialyzed against 0.9% NaCl, and concentrated to 1 or 4 mg/ml using centrifugal concentrators before analysis. Plasmid DNA having 5921 bp was comprised of kanamycin resistance and luciferase genes, elongation factor-1 a promoter and first intron, CMV enhancer, RU5 translational enhancer from HTLV I, SV40 late polyadenylation site, and ColE1 origin of replication was used.

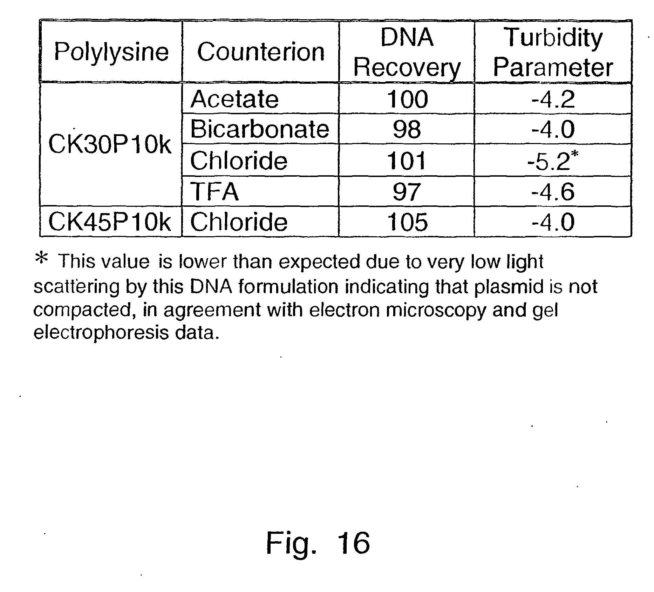

Colloidal stability for the DNA complexes was determined by measuring sedimentation of condensed DNA during centrifugation (3,400 for 1 min) and scattering of light (turbidity) in the wavelength range of 330-415 nm. The turbidity parameter is the slope of a straight line obtained by plotting log of apparent absorbance (due to scattering) vs. log of incident wavelength in a range outside the true absorption by DNA or peptides (330-415 nm). According to the Rayleigh law, particles that are small compared to the wavelength of light should have Turbidity Parameter of - 4. Larger particles, however, scatter light differently and have Turbidity Parameters in the range of ~ -1 to - 3. Very large aggregates, have a Turbidity Parameter of ~ -1. We have found that all the tested DNA formulations were colloidally stable in normal saline (0.9% NaCl) as judged by sedimentation and turbidity measurements. We also found that the ability of polylysines to condense DNA depends on type of associated counterions and length of polylysine. CK30P10k with chloride represents the extreme case since it does not condense DNA or condenses it very poorly. (Fig. 16).

### Example 11

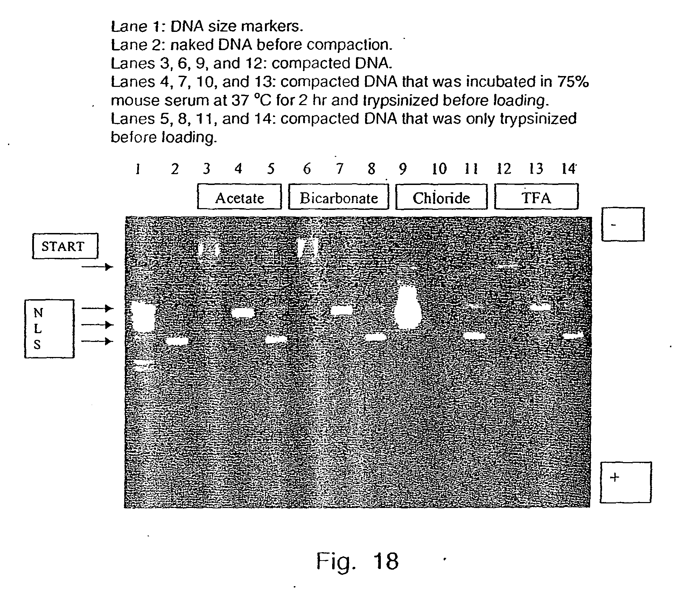

DNA compacted by CK30P10K with various counterions was electrophoresed through an agarose gel to examine the effect of counterion on net charge of condensed DNA. DNA samples were loaded directly on the gel (1.5 µg) or after trypsin treatment for 40 min (0.2 µg) to remove polylysine and visualize DNA integrity and relative quantities of supercoiled, nicked, and linear plasmid forms. DNA either migrated to the cathode (CK30/acetate, CK30/bicarbonate, CK45/chloride), remained in the well (CK30/TFA), or migrated to the anode (CK30/chloride). (Fig. 18). Therefore, counterions influence effective net charge of condensed DNA as visualized by gel electrophoresis. Acetate and bicarbonate bound to CK30P10k and chloride bound to CK45P10k result in slightly positive net charge, while TFA results in electrically neutral complexes.

Serum stability was also evaluated for each of the compacted DNA complexes. This was assessed by incubating DNA samples with 75% mouse serum at 37°C for 2 hr, removing polylysine by trypsinization, and evaluating DNA integrity by gel electrophoresis. Under these conditions, properly condensed DNA is stable, although some nicking and linearization (very little) occurs. Naked DNA, on the other hand, is completely digested within a few minutes (Fig. 18). We found that the ability of polylysines to condense and protect DNA depends on type of associated counterions and length of polylysine. CK30P10k with chloride again represents the extreme case since it does not condense DNA or condenses it very poorly and does not protect against nucleases.

### Example 12

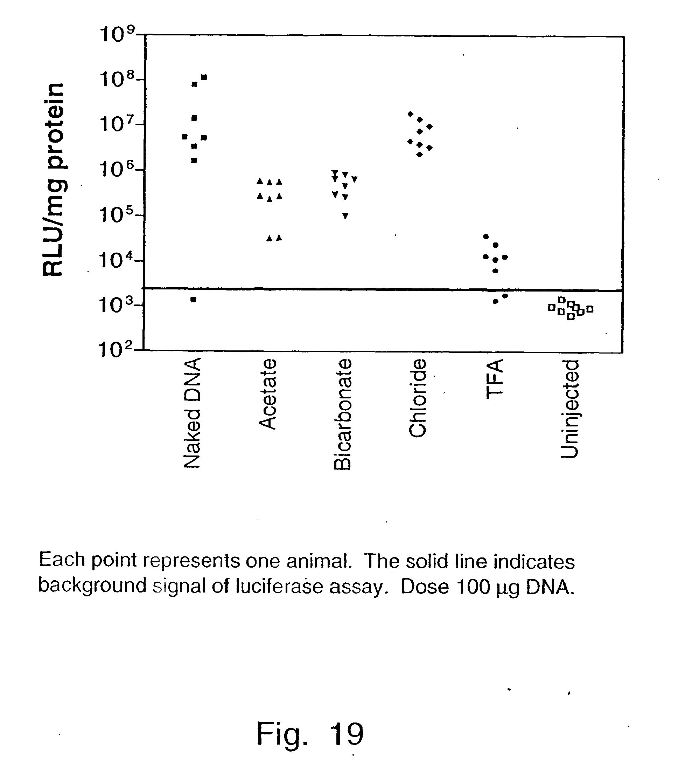

Intramuscular gene delivery was assessed for each of the counterion forms of CK30P10K. Fifty µl of DNA was injected into quadriceps of each leg of CD-1 mice (4-6 weeks old). The total dose was 100 µg. Prior to the injection, the animals were anesthetized by intraperitoneal injection of a rodent cocktail of Ketamine, Xylazine, and Acepromazine. One day after the injection, the mice were terminated and entire quadrceps removed and processed. Protein and luciferase activity were determined. (Fig. 19).

The morphology of the compacted DNA complexes appears to have influenced their *in vivo* transfection efficiency. CK30/TFA gave the lowest expression (RLU/mg protein), CK30/acetate and CK30/bicarbonate (more relaxed structures) gave 10-100-fold higher RLU/mg, and CK30/chloride gave the expression at the level of naked DNA (same as or 10-fold higher than CK30/acetate, depending on harvest day). We have found that naked DNA is more efficient than condensed DNA and the TFA formulation is much less efficient than other forms of condensed DNA for intramuscular gene delivery.

### Example 13

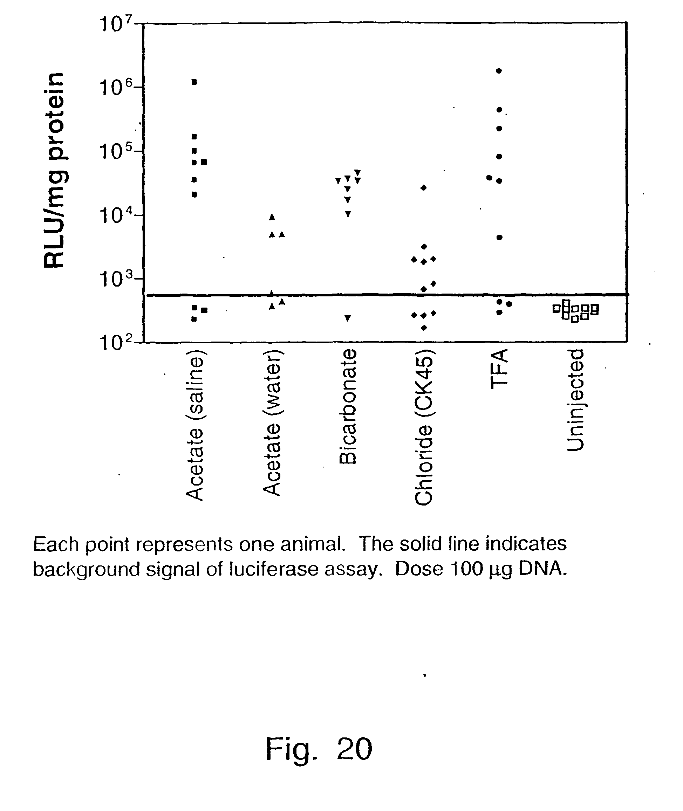

Intranasal gene delivery was assessed for each of the counterion forms of CK30P10K. Twenty five µl of DNA was administered in 5-µl aliquots into nostrils of C57/BL6 mice using an automated pipette. The total dose was 100 µg. Prior to the injection, the animals were anesthetized by intraperitoneal injection of a rodent cocktail of Ketamine, Xylazine, and Acepromazine. Two days after the injection, the mice were terminated and entire lungs removed and processed. Protein and luciferase activity were determined (Fig. 20). In intranasal application, the acetate, bicarbonate, and TFA formulations of condensed DNA are the most efficient among the tested formulations, and naked DNA and CK45/chloride were much less effective. We also found that condensed DNA administered intranasally in water is about 10-fold less efficient than the same DNA administered in saline.

### LITERATURE CITED

1. Cooper, M.J.. (1996) Non-infectious gene transfer and expression systems for cancer gene therapy.
2. Semin.Oncol. 23:172-188 Weiss, R. and Nelson, D. Washington Post, 9/29/99, page A1.
3. Takeshita, S., Gai, D., Leclerc, G., Pickering, J.G., Riesssen, R., Wier, L., and Isner, J.M. (1994) Increased gene expression after liposome-mediated arterial gene transfer associated with intimal smooth muscle cell proliferation. J. Clin. Invest. 93:652-661.
4. Zabner, J., Fasbender, A.J., Moninger, T., Poellinger, D.A., and Welsh, M.J. (1995) Cellular and molecular barriers to gene transfer by a cationic lipid. J. Biol. Chem. 270:18997-19007.
5. Wilke, M., Fortunati, E., van den Broek, M., Hoogeveen, A.T., and Scholte, B.J. (1996) Efficacy of a peptide-based gene delivery system depends on mitotic activity. Gene Ther. 3:1133-1142.
6. Fasbender, A., Zabner, J., Zeiher, B.G., and Welsh, M.J. (1997) A low rate of cell proliferation and reduce DNA uptake limit cationic lipid-mediated gene transfer to primary cultures of ciliated human airway epithelia. Gene Ther. 41173-1180.
7. Sebestyen, M.G., Ludtke, J.J., Bassik, M.C., Zhang, G., Budker, V., Lukhtanov, E.A., Hagstrom, J.E., and Wolff. J.A. (1998) DNA vector chemistry: the covalent attachment of signal peptides to plasmid DNA. Nat. Biotechnol. 16:80-85.
8. Jiang, C., O'Connor, S.P., Fang, S.L., Wang, K.X., Marshall, J., Williams, J.L., Wilburn, B., Echelard, Y., and Cheng, S. (1998) Efficiency of cationic lipid-mediated transfection of polarized and differentiated airway epithelial cells in vitro and in vivo.
9. Tseng, W.C., Haselton, F.R., and Giorgio, T.D. (1999) Mitosis enhances transgene expression of plasmid delivered by cationic liposomes. Biochim. Biophy. Acta 1445:53-64.
10. Mortimer, J., Tam, P., MacLachlan, I., Graham, R.W., Saravolac, E.G., and Joshi, P.B. (1999) Cationic lipid-mediated transfection of cells in culture requires mitotic activity. Gene Ther. 6:403-411.
11. Mirzayans, R., Aubin, R., and Paterson, M. (1992) Differential expression and stability of foreign genes introduced into human fibroblasts by nuclear versus cytoplasmic microinjection. Mutat. Res. 281:115-122.
12. Dworetzky, S.I. and Feldherr, C.M. (1988) Translocation of RNA-coated gold particles through the nuclear pores of oocytes. J. Cell Biol. 106:575-584.
13. Feldherr, C.M. and Akin D. (1991) Signal-mediated nuclear transport in proliferating and growth-arrested BALB/c 3T3 cells. J. Cell Biol. 115:933-939.

## Claims

1. A non-naturally occurring composition comprising unaggregated nucleic acid complexes, each complex consisting essentially of a single nucleic acid molecule and one or more polycation molecules wherein said complexes are formed by mixing said nucleic acid molecule and said polycation molecules, wherein prior to mixing said polycation molecules have a counterion selected from the group consisting of acetate, bicarbonate, and chloride, wherein said complexes are rod-shaped when visualized by transmission electron microscopy.

2. The composition of claim 1, wherein the polycation molecules are polylysine or a polylysine derivative.

3. The composition of claim 2, wherein the polylysine derivative is polylysine peptide with a cysteine residue.

4. The composition of claim 1, said rod-shaped complexes have a length of 100-300 nm when visualized by transmission electron microscopy.

5. The composition of claim 1, wherein the rod-shaped complexes have a length of 100-200 nm when visualized by transmission electron microcopy.

6. The composition of claim 1, wherein the rod-shaped complexes have a diameter of 10-20 nm when visualized by transmission electron microscopy.

7. The composition of claim 1, wherein the rod-shaped complexes have a length of 100-300 nm and a diameter of 10-20 nm when visualized by transmission electron microscopy.

8. The composition of claim 1, wherein said single nucleic acid molecule encodes at least one functional protein.

9. A method of preparing a composition according to any one of claims 1 to 3 which comprises adding the nucleic acid to the polycation having acetate as a counterion, at a salt concentration sufficient for compaction of the complex.

10. A method of preparing a composition comprising unaggregated nucleic acid complexes, each complex consisting essentially of a single nucleic acid molecule and one or more polycation molecules, said method comprising:
adding a nucleic acid molecule to a polycation molecule while mixing to form a mixture, said polycation molecule having a counterion selected from the group consisting of acetate, bicarbonate, and chloride whereby unaggregated nucleic acid complexes are formed, wherein each complex consists essentially of a single nucleic acid molecule and one or more polycation molecules.

11. The method of claim 9 or claim 10, in which the mixing is monitored to detect, prevent or correct, the formation of aggregated or relaxed complexes.

12. The method of claim 9, wherein the salt is NaCl.

13. The method of claim 9 or claim 10, wherein the nucleic acid and the polycation are each, at the time of mixing, in a solution having a salt concentration of 0.05 to 1.5 M.

14. The method of claim 9 or claim 10, in which the molar ratio of the phosphate groups of the nucleic acid to the positively charged groups of the polycation is in the range of 4:1 to 1:4.

15. The method of claim 9 or claim 10, in which the nucleic acid is added to the polycation, while vortexing at high speed.

16. The method of claim 9 or claim 10, wherein the mixing is monitored by a method selected from the group consisting of electron microscopy, light scattering, circular dichroism, and absorbance measurement.

17. The method of claim 10, wherein the polycation molecules are polylysine or a polylysine derivative.

18. The method of claim 17, wherein the polylysine derivative is polylysine peptide with a cysteine residue.

19. The non-naturally occurring composition of claim 1, wherein said polycation molecule has a nucleic acid binding moiety through which it is complexed to the nucleic acid, and wherein said nucleic acid molecule encodes at least one functional protein.

20. The composition of claim 19, wherein the polycation molecules are polylysine or a polylysine derivative.

21. The composition of claim 20, wherein the polylysine derivative is polylysine peptide with a cysteine residue.

22. The non-naturally occurring composition of claim 19, wherein said nucleic acid molecule comprises a promoter which controls transcription of an RNA molecule encoding the functional protein.

23. The non-naturally occurring composition of claim 19, wherein the protein is therapeutic.

24. The non-naturally occurring composition of claim 1, wherein the nucleic acid complex is compacted to a diameter not more than 12 nm.

25. The non-naturally occurring composition of claim 1, wherein each complex consists essentially of a single double-stranded cDNA molecule and one or more polycation molecules.

26. The non-naturally occurring composition of claim 1, wherein said nucleic acid molecule encodes at least one antisense nucleic acid.

27. The non-naturally occurring composition of claim 1, wherein said nucleic acid molecule is an RNA molecule.

28. The method of preparing a composition of claim 10, wherein said mixing is performed in the absence of added salt.

29. The composition of claim 25, 26 or 27, or the method of claim 28, wherein the polycation molecules are polylysine or a polylysine derivative.

30. The composition of claim 25, 26 or 27, or the method of claim 28, wherein the polylysine derivative is polylysine peptide with a cysteine residue.

31. Non-naturally occurring, soluble compacted complexes of a nucleic acid and a polycation molecule made by the method of any one of claims 9, 10, and 28.

32. The complexes of claim 31, wherein the polycation molecules are polylysine or a polylysine derivative.

33. The complexes of claim 32, wherein the polylysine derivative is polylysine peptide with a cysteine residue

34. Use of the composition according to claim 1 in the manufacture of a medicament for preventing or treating a disease or other clinical condition in a subject, wherein the medicament is for administration intramuscularly or to the lung of the subject
said nucleic acid being one whose integration, hybridization or expression within target cells of said subject prevents or treats said disease or other clinical condition.

35. The use of claim 34, wherein the disease or other clinical condition is selected from pulmonary diseases, metabolic disorders and cancers.

36. The use of claim 34, wherein the disease or other clinical condition is cystic fibrosis.

37. The use of claim 34, wherein the medicament is for administration by inhalation.

38. The use of claim 34, wherein the medicament is for administration by intramuscular injection.

39. The use of claim 34, wherein the polycation molecules are polylysine or a polylysine derivative.

40. The use of claim 39, wherein the polylysine derivative is polylysine peptide with a cysteine residue.

41. The composition of claim 1, wherein the nucleic acid complexes are associated with a lipid.

42. The composition of any one of claims 25, 26 and 27 wherein the nucleic acid complex is compacted to a diameter not more than 12 nm.

43. The method of claim 28, in which the mixing is monitored to detect, prevent or correct, the formation of aggregated or relaxed complexes.

44. The method of claim 28, in which the molar ratio of the phosphate groups of the nucleic acid to the positively charged groups of the polycation is in the range of 4:1 to 1:4.

45. The method of claim 28, in which the nucleic acid is added to the polycation, while vortexing at high speed.

46. The method of claim 28, wherein the mixing is monitored by a method selected from the group consisting of electron microscopy, light scattering, circular dichroism, and absorbance measurement.

47. The composition of claim 1, wherein said polycation is CK15-60P10 and the counterion is acetate, wherein CK15-60P10 is a polyamino acid polymer of one N-terminal cysteine and 15-60 lysine residues, wherein a molecule of polyethylene glycol having an average molecular weight of 10 kDa is attached to the cysteine residue.

48. The composition of claim 19, wherein the polycation is CK15-60P10, and the counterion is acetate, wherein CK15-60 is a polyamino acid polymer of one N-terminal cysteine and 15-60 lysine residues, wherein a molecule of polyethylene glycol having an average molecular weight of 10 kDa is attached to the cysteine residue.

49. The composition of claim 25, wherein said polycation is CK15-60P10, and said counterion is acetate, wherein CK15-60P10 is a polyamino acid polymer of one N-terminal cysteine and 15-60 lysine residues, wherein a molecule of polyethylene glycol having an average molecular weight of 10 kDa is attached to the cysteine residue.

50. The composition of claim 26, wherein said polycation is CK15-60P10, and the counterion is acetate, wherein CK15-60P10 is a polyamino acid polymer of one N-terminal cysteine and 15-60 lysine residues, wherein a molecule of polyethylene glycol having an average molecular weight of 10 kDa is attached to the cysteine residue.

51. The composition of claim 27, wherein said polycation is CK15-60P10, and said counterion is acetate, wherein CK15-60P10 is a polyamino acid polymer of one N-terminal cysteine and 15-60 lysine residues, wherein a molecule of polyethylene glycol having an average molecular weight of 10 kDa is attached to the cysteine residue.

52. The method of claim 28, wherein said polycation is CK15-60P10, and said counterion is acetate, wherein CK15-60P10 is a polyamino acid polymer of one N-terminal cysteine and 15-60 lysine residues, wherein a molecule of polyethylene glycol having an average molecular weight of 10 kDa is attached to the cysteine residue.

53. The composition of any one of claims 47 to 51, wherein the polycation molecule comprises 30 residues of lysine.

54. The composition of any one of claims 47 to 51, wherein the polycation molecule comprises a targeting moiety.

55. The composition of claim 47 or claim 48, wherein the nucleic acid complex is compacted to a diameter not more than 12 nm.

56. The composition of any one of claims 47 to 51, which is lyophilized.

57. The composition of claim 47, which is rehydrated after lyophilization.

58. The composition of any one of claims 47 to 51, which does not contain a disaccharide.

59. An *in vitro* method of delivering polynucleotides to cells comprising:
contacting the composition of claim 57 with cells, whereby the nucleic acid is delivered to and taken up by the cells.

60. The method of claim 59, wherein the composition does not contain a disaccharide.

61. The non-naturally occurring composition of claim 48, wherein said nucleic acid molecule comprises a promoter which controls transcription of an RNA molecule encoding the functional protein.

62. The non-naturally occurring composition of claim 48, wherein the protein is therapeutic.

63. The composition of claim 48 which is rehydrated after lyophilization.

64. An *in vitro* method of delivering polynucleotides to cells comprising:
contacting the composition of claim 63 with cells, whereby the protein is expressed.

65. The composition of claim 49 which is rehydrated after lyophilization.

66. An *in vitro* method of delivering polynucleotides to cells comprising:
contacting the composition of claim 65 with cells, wherein the polynucleotide encodes a protein and whereby the protein is expressed.

67. The composition of claim 50 which is rehydrated after lyophilization.

68. The composition of claim 67, which does not contain a disaccharide.

69. The composition of claim 51 which is lyophilized and rehydrated.

70. An *in vitro* method of delivering polynucleotides to cells comprising:
contacting the composition of claim 69 with cells, whereby the polynucleotide is delivered to and taken up by the cells.

71. The method of claim 52, further comprising lyophilizing the unaggregated nucleic acid complexes.

72. The method of claim 71, further comprising rehydrating the lyophilized nucleic acid complexes.

## Patentansprüche

1. Eine nicht natürlich vorkommende Zusammensetzung, umfassend nicht aggregierte Nukleinsäurekomplexe, wobei jeder Komplex im Wesentlichen aus einem einzigen Nukleinsäuremolekül und einem oder mehreren Polykationmolekülen besteht, wobei die Komplexe durch Mischen des Nukleinsäuremoleküls und der Polykationmoleküle gebildet werden, wobei vor dem Mischen die Polykationmoleküle ein Gegenion haben, ausgewählt aus der Gruppe bestehend aus Acetat, Bicarbonat und Chlorid, wobei die Komplexe stäbchenförmig sind, wenn sie mittels Transmissionselektronenmikroskopie sichtbar gemacht werden.

2. Zusammensetzung nach Anspruch 1, wobei die Polykationmoleküle Polylysin oder ein Polylysinderivat sind.

3. Zusammensetzung nach Anspruch 2, wobei das Polylysinderivat ein Polylysinpeptid mit einem Cysteinrest ist.

4. Zusammensetzung nach Anspruch 1, wobei die stäbchenförmigen Komplexe eine Länge von 100-300 nm haben, wenn sie mittels Transmissionselektronenmikroskopie sichtbar gemacht werden.

5. Zusammensetzung nach Anspruch 1, wobei die stäbchenförmigen Komplexe eine Länge von 100-200 nm haben, wenn sie mittels Transmissionselektronenmikroskopie sichtbar gemacht werden.

6. Zusammensetzung nach Anspruch 1, wobei die stäbchenförmigen Komplexe einen Durchmesser von 10-20 nm haben, wenn sie mittels Transmissionselektronenmikroskopie sichtbar gemacht werden.

7. Zusammensetzung nach Anspruch 1, wobei die stäbchenförmigen Komplexe eine Länge von 100-300 nm und einen Durchmesser von 10-20 nm haben, wenn sie mittels Transmissionselektronenmikroskopie sichtbar gemacht werden.

8. Zusammensetzung nach Anspruch 1, wobei das einzelne Nukleinsäuremolekül wenigstens ein funktionelles Protein kodiert.

9. Verfahren zur Herstellung einer Zusammensetzung nach einem der Ansprüche 1 bis 3, umfassend Hinzufügen der Nukleinsäure zu dem Polykation, welches Acetat als Gegenion hat, bei einer für die Verdichtung des Komplexes ausreichenden Salzkonzentration.

10. Verfahren zur Herstellung einer Zusammensetzung umfassend nicht aggregierte Nukleinsäurekomplexe, wobei jeder Komplex im Wesentlichen aus einem einzigen Nukleinsäuremolekül und einem oder mehreren Polykationmolekülen besteht, und wobei das Verfahren umfasst:
Hinzufügen eines Nukleinsäuremoleküls zu einem Polykationmolekül während eines Mischvorgangs um eine Mischung zu bilden, wobei das Polykationmolekül ein Gegenion hat, ausgewählt aus der Gruppe bestehend aus Acetat, Bicarbonat und Chlorid, wodurch nicht aggregierte Nukleinsäurekomplexe gebildet werden, und wobei jeder Komplex im Wesentlichen aus einem einzigen Nukleinsäuremolekül und einem oder mehreren Polykationmolekülen besteht.

11. Verfahren nach Anspruch 9 oder Anspruch 10, wobei das Mischen überwacht wird, um die Bildung von aggregierten oder entspannten Komplexen zu detektieren, zur verhindern oder zu korrigieren.

12. Verfahren nach Anspruch 9, wobei das Salz NaCl ist.

13. Verfahren nach Anspruch 9 oder Anspruch 10, wobei die Nukleinsäure und das Polykation sich zum Zeitpunkt des Mischens jeweils in einer Lösung befinden, deren Salzkonzentration 0,05 bis 1,5 M beträgt.

14. Verfahren nach Anspruch 9 oder Anspruch 10, wobei das Molverhältnis der Nukleinsäurephosphatgruppen zu den positiv geladenen Gruppen des Polykations im Bereich von 4:1 bis 1:4 ist.

15. Verfahren nach Anspruch 9 oder Anspruch 10, wobei die Nukleinsäure während Vortexen bei hoher Geschwindigkeit zu dem Polykation hinzugefügt wird.

16. Verfahren nach Anspruch 9 oder Anspruch 10, wobei das Mischen mittels eines Verfahrens, ausgewählt aus der Gruppe bestehend aus Elektronenmikroskopie, Lichtstreuung, Circulardichroismus und Absorptionsmessung, überwacht wird.

17. Verfahren nach Anspruch 10, wobei die Polykationmoleküle Polylysin oder ein Polylysinderivat sind.

18. Verfahren nach Anspruch 17, wobei das Polylysinderivat ein Polylysinpeptid mit einem Cysteinrest ist.

19. Nicht natürlich vorkommende Zusammensetzung nach Anspruch 1, wobei das Polykationmolekül einen nukleinsäurebindenen Rest hat, worüber es an die Nukleinsäure komplexiert ist und wobei das Nukleinsäuremolekül wenigstens ein funktionelles Protein kodiert.

20. Zusammensetzung nach Anspruch 19, wobei die Polykationmoleküle Polylysin oder ein Polylysinderivat sind.

21. Zusammensetzung nach Anspruch 20, wobei das Polylysinderivat ein Polylysinpeptid mit einem Cysteinrest ist.

22. Nicht natürlich vorkommende Zusammensetzung nach Anspruch 19, wobei das Nukleinsäuremolekül einen Promotor umfasst, der die Transkription eines RNA-Moleküls kontrolliert, welches das funktionelle Protein kodiert.

23. Nicht natürlich vorkommende Zusammensetzung nach Anspruch 19, wobei das Protein therapeutisch wirksam ist.

24. Nicht natürlich vorkommende Zusammensetzung nach Anspruch 1, wobei der Nukleinsäurekomplex auf einen Durchmesser von nicht mehr als 12 nm verdichtet ist.

25. Nicht natürlich vorkommende Zusammensetzung nach Anspruch 1, wobei jeder Komplex im Wesentlichen aus einem einzigen doppelsträngigen cDNA-Molekül und einem oder mehreren Polykationmolekülen besteht.

26. Nicht natürlich vorkommende Zusammensetzung nach Anspruch 1, wobei das Nukleinsäuremolekül wenigstens eine antisense-Nukleinsäure kodiert.

27. Nicht natürlich vorkommende Zusammensetzung nach Anspruch 1, wobei das Nukleinsäuremolekül ein RNA-Molekül ist.

28. Verfahren zur Herstellung einer Zusammensetzung nach Anspruch 10, wobei das Mischen in Abwesenheit von hinzugefügtem Salz durchgeführt wird.

29. Zusammensetzung nach Anspruch 25, 26 oder 27, oder das Verfahren nach Anspruch 28, wobei die Polykationmoleküle Polylysin oder ein Polylysinderivat sind.

30. Zusammensetzung nach Anspruch 25, 26 oder 27, oder das Verfahren nach Anspruch 28, wobei das Polylysinderivat ein Polylysinpeptid mit einem Cysteinrest ist.

31. Nicht natürlich vorkommende, löslich verdichtete Komplexe aus einer Nukleinsäure und einem Polykationmolekül, hergestellt mittels eines Verfahrens nach einem der Ansprüche 9, 10, und 28.

32. Komplexe nach Anspruch 31; wobei die Polykationmoleküle Polylysin oder ein Polylysinderivat sind.

33. Komplexe nach Anspruch 32, wobei das Polylysinderivat ein Polylysinpeptid mit einem Cysteinrest ist.

34. Verwendung der Zusammensetzung nach Anspruch 1 zur Herstellung eines Medikamentes zur Vorbeugung oder Behandlung einer Krankheit oder eines anderen klinischen Zustandes bei einem Subjekt, wobei das Medikament intramuskulär oder in die Lunge des Subjekts verabreicht wird, und wobei
die Nukleinsäure eine Nukleinsäure ist, deren Integration, Hybridisierung oder Expression in den Zielzellen des Subjekts der Krankheit oder einem anderen klinischen Zustand vorbeugt oder diese behandelt.

35. Verwendung nach Anspruch 34, wobei die Krankheit oder ein anderer klinischer Zustand ausgewählt ist aus Lungenerkrankungen, Stoffwechselstörungen und Krebs.

36. Verwendung nach Anspruch 34, wobei die Krankheit oder ein anderer klinischer Zustand zystische Fibrose ist.

37. Verwendung nach Anspruch 34, wobei das Medikament durch Inhalation verabreicht wird.

38. Verwendung nach Anspruch 34, wobei das Medikament über eine intramuskuläre Injektion verabreicht wird.

39. Verwendung nach Anspruch 34, wobei die Polykationmoleküle Polylysin oder ein Polylysinderivat sind.

40. Verwendung nach Anspruch 39, wobei das Polylysinderivat ein Polylysinpeptid mit einem Cysteinrest ist.

41. Zusammensetzung nach Anspruch 1, wobei die Nukleinsäurekomplexe mit einem Lipid assoziiert sind.

42. Zusammensetzung nach einem der Ansprüche 25, 26 und 27, wobei der Nukleinsäurekomplex auf einen Durchmesser von nicht mehr als 12 nm verdichtet ist.

43. Verfahren nach Anspruch 28, wobei das Mischen überwacht wird, um die Bildung von aggregierten oder entspannten Komplexen zu detektieren, zu verhindern oder zu korrigieren.

44. Verfahren nach Anspruch 28, wobei das Molverhältnis der Nukleinsäurephosphatgruppen zu den positiv geladenen Gruppen des Polykations im Bereich von 4:1 bis 1:4 ist.

45. Verfahren nach Anspruch 28, wobei die Nukleinsäure während Vortexen bei hohen Geschwindigkeiten zu dem Polykation hinzugefügt wird.

46. Verfahren nach Anspruch 28, wobei das Mischen mittels eines Verfahrens ausgewählt aus der Gruppe bestehend aus Elektronenmikroskopie, Lichtstreuung, Circulardichroismus und Absorptionsmessung überwacht wird.

47. Zusammensetzung nach Anspruch 1, wobei das Polykation CK15-60P10 und das Gegenion Acetat ist, wobei CK15-60P10 eine Polyaminosäurepolymer aus einem N-terminalen Cystein und 15-60 Lysinresten ist, wobei ein Polyethylenglykolmolekül mit einer durchschnittlichen Molekülmasse von 10 kDa mit dem Cysteinrest verknüpft ist.

48. Zusammensetzung nach Anspruch 19, wobei das Polykation CK15-60P10 und das Gegenion Acetat ist, wobei CK15-60P10 eine Polyaminosäurepolymer aus einem N-terminalen Cystein und 15-60 Lysinresten ist, wobei ein Polyethylenglykolmolekül mit einer durchschnittlichen Molekülmasse von 10 kDa mit dem Cysteinrest verknüpft ist.

49. Zusammensetzung nach Anspruch 25, Zusammensetzung nach Anspruch 1, wobei das Polykation CK15-60P10 und das Gegenion Acetat ist, wobei CK15-60P10 eine Polyaminosäurepolymer aus einem N-terminalen Cystein und 15-60 Lysinresten ist, wobei ein Polyethylenglykolmolekül mit einer durchschnittlichen Molekülmasse von 10 kDa mit dem Cysteinrest verknüpft ist.

50. Zusammensetzung nach Anspruch 26, wobei das Polykation CK15-60P10 und das Gegenion Acetat ist, wobei CK15-60P10 eine Polyaminosäurepolymer aus einem N-terminalen Cystein und 15-60 Lysinresten ist, wobei ein Polyethylenglykolmolekül mit einer durchschnittlichen Molekülmasse von 10 kDa mit dem Cysteinrest verknüpft ist.

51. Zusammensetzung nach Anspruch 27, wobei das Polykation CK15-60P10 und das Gegenion Acetat ist, wobei CK15-60P10 eine Polyaminosäurepolymer aus einem N-terminalen Cystein und 15-60 Lysinresten ist, wobei ein Polyethylenglykolmolekül mit einer durchschnittlichen Molekülmasse von 10 kDa mit dem Cysteinrest verknüpft ist.

52. Zusammensetzung nach Anspruch 28, wobei das Polykation CK15-60P10 und das Gegenion Acetat ist, wobei CK15-60P10 eine Polyaminosäurepolymer aus einem N-terminalen Cystein und 15-60 Lysinresten ist, wobei ein Polyethylenglykolmolekül mit einer durchschnittlichen Molekülmasse von 10 kDa mit dem Cysteinrest verknüpft ist.

53. Zusammensetzung nach einem der Ansprüche 47 bis 51, wobei das Polykationmolekül 30 Lysinreste umfasst.

54. Zusammensetzung nach einem der Ansprüche 47 bis 51, wobei das Polykationmolekül einen Zielrest umfasst.

55. Zusammensetzung nach Anspruch 47 oder Anspruch 48, wobei der Nukleinsäurekomplex auf einen Durchmesser von nicht mehr als 12 nm verdichtet ist.

56. Zusammensetzung nach einem der Ansprüche 47 bis 51, wobei die Zusammensetzung lyophilisiert ist.

57. Zusammensetzung nach Anspruch 47, wobei die Zusammensetzung nach Lyophilisierung rehydratisiert wird.

58. Zusammensetzung nach einem der Ansprüche 47 bis 51, wobei die Zusammensetzung kein Disaccharid enthält.

59. *in-vitro*-Verfahren zur Abgabe von Polynukleotiden an Zellen umfassend:
In-Kontakt-bringen der Zusammensetzung nach Anspruch 57 mit Zellen, wodurch die Nukleinsäure an die Zellen abgegeben und von diesen aufgenommen wird.

60. Verfahren nach Anspruch 59, wobei die Zusammensetzung kein Disaccharid enthält.

61. Nicht natürlich vorkommende Zusammensetzung nach Anspruch 48, wobei das Nukleinsäuremolekül einen Promotor umfasst, der die Transkription eines RNA-Moleküls kontrolliert, welches das funktionelle Protein kodiert.

62. Nicht natürlich vorkommende Zusammensetzung nach Anspruch 48, wobei das Protein therapeutisch wirksam ist.

63. Zusammensetzung nach Anspruch 48, wobei die Zusammensetzung nach Lyophilisierung rehydratisiert wird.

64. *in-vitro*-Verfahren zur Abgabe von Polynukleotiden an Zellen, umfassend:
In-Kontakt-bringen der Zusammensetzung nach Anspruch 63 mit Zellen, wodurch das Protein exprimiert wird.

65. Zusammensetzung nach Anspruch 49, wobei die Zusammensetzung nach Lyophilisierung rehydratisiert wird.

66. *in*-*vitro*-Verfahren zur Abgabe von Polynukleotiden an Zellen, umfassend:
In-Kontakt-bringen der Zusammensetzung nach Anspruch 65 mit Zellen, wobei das Polynukleotid ein Protein kodiert und wodurch das Protein exprimiert wird.

67. Zusammensetzung nach Anspruch 50, wobei die Zusammensetzung nach Lyophilisierung rehydratisiert wird.

68. Zusammensetzung nach Anspruch 67, wobei die Zusammensetzung kein Disaccharid enthält.

69. Zusammensetzung nach Anspruch 51, wobei die Zusammensetzung lyophilisiert und rehydratisiert wird.

70. *in-vitro*-Verfahren zur Abgabe von Polynukleotiden an Zellen umfassend:
In-Kontakt-bringen der Zusammensetzung nach Anspruch 69 mit Zellen, wodurch das Polynukleotid an die Zellen abgegeben und von diesen aufgenommen wird.

71. Verfahren nach Anspruch 52, weiterhin umfassend Lyophilisieren der nicht aggregierten Nukleinsäurekomplexe.

72. Verfahren nach Anspruch 71, weiterhin umfassend Rehydratisieren der lyophilisierten Nukleinsäurekomplexe.

## Revendications

1. Composition non naturelle comprenant des complexes d'acide nucléique non agglomérés, chaque complexe consistant essentiellement en une molécule d'acide nucléique unique et une ou plusieurs molécules de polycation dans lesquelles lesdits complexes sont formés en mélangeant ladite molécule d'acide nucléique et lesdites molécules de polycation, dans lesquelles, avant le mélange, lesdites molécules de polycation ont un contre-ion choisi dans le groupe constitué d'acétate, de bicarbonate et de chlorure, dans laquelle lesdits complexes sont bacilliformes quand ils sont visualisés par microscopie électronique par transmission.

2. Composition selon la revendication 1, dans laquelle les molécules de polycation sont de la polylysine ou un dérivé de polylysine.

3. Composition selon la revendication 2, dans laquelle le dérivé de polylysine est un peptide de polylysine avec un résidu de cystéine.

4. Composition selon la revendication 1, lesdits complexes bacilliformes ayant une longueur de 100-300 nm lorsqu'ils sont visualisés par microscopie électronique par transmission.

5. Composition selon la revendication 1, dans laquelle les complexes bacilliformes ont une longueur de 100-200 nm quand ils sont visualisés par microscopie électronique par transmission.

6. Composition selon la revendication 1, dans laquelle les complexes bacilliformes ont un diamètre de 10-20 nm quand ils sont visualisés par microscopie électronique par transmission.

7. Composition selon la revendication 1, dans laquelle les complexes bacilliformes ont une longueur de 100-300 nm et un diamètre de 10-20 nm quand ils sont visualisés par microscopie électronique par transmission.

8. Composition selon la revendication 1, dans laquelle ladite molécule d'acide nucléique unique code au moins une protéine fonctionnelle.

9. Procédé de préparation d'une composition selon l'une quelconque des revendications 1 à 3, qui comprend l'addition de l'acide nucléique au polycation ayant de l'acétate comme contre-ion, à une concentration de sel suffisante pour l'agglomération du complexe.

10. Procédé de préparation d'une composition comprenant des complexes d'acide nucléique non agglomérés, chaque complexe consistant essentiellement en une molécule d'acide nucléique unique et une ou plusieurs molécules de polycation, ledit procédé comprenant :
l'addition d'une molécule d'acide nucléique à une molécule de polycation tout en mélangeant pour former un mélange, ladite molécule de polycation ayant un contre-ion choisi dans le groupe constitué d'acétate, de bicarbonate, et de chlorure, moyennant quoi des complexes d'acide nucléique non agglomérés sont formés, dans lesquels chaque complexe consiste essentiellement en une molécule d'acide nucléique unique et une ou plusieurs molécules de polycation.

11. Procédé selon la revendication 9 ou la revendication 10, dans lequel le mélange est contrôlé pour détecter, empêcher ou corriger, la formation de complexes agglomérés ou relâchés.

12. Procédé selon la revendication 9, dans lequel le sel est du NaCl.

13. Procédé selon la revendication 9 ou la revendication 10, dans lequel l'acide nucléique et le polycation sont chacun, au moment du mélange, dans une solution ayant une concentration de sel de 0,05 à 1,5 M.

14. Procédé selon la revendication 9 ou la revendication 10, dans lequel le rapport molaire des groupes phosphate de l'acide nucléique aux groupes positivement chargés du polycation est dans la gamme allant de 4 :1 à 1 :4.

15. Procédé selon les revendications 9 ou 10, dans lequel l'acide nucléique est ajouté au polycation, tout en l'agitant par vortex à grande vitesse.

16. Procédé selon les revendications 9 ou 10, dans lequel le mélange est contrôlé par un procédé choisi dans le groupe constitué de la microscopie électronique, la diffusion de lumière, le dichroïsme circulaire, et la mesure d'absorption.

17. Procédé selon la revendication 10, dans lequel les molécules de polycation sont la polylysine ou un dérivé de polylysine.

18. Procédé selon la revendication 17, dans lequel le dérivé de polylysine est un peptide de polylysine avec un résidu de cystéine.

19. Composition non naturelle selon la revendication 1, dans laquelle ladite molécule de polycation a une portion de liaison d'acide nucléique à travers laquelle elle est complexée à l'acide nucléique et dans laquelle ladite molécule d'acide nucléique code au moins une protéine fonctionnelle.

20. Composition selon la revendication 19, dans laquelle les molécules de polycation sont la polylysine ou un dérivé de polylysine.

21. Composition selon la revendication 20, dans laquelle le dérivé de polylysine est un peptide de polylysine avec un résidu de cystéine.

22. Composition non naturelle selon la revendication 19, dans laquelle ladite molécule d'acide nucléique comprend un promoteur qui commande la transcription d'une molécule d'ARN codant la protéine fonctionnelle.

23. Composition non naturelle selon la revendication 19, dans laquelle la protéine est thérapeutique.

24. Composition non naturelle selon la revendication 1, dans laquelle le complexe d'acide nucléique est compacté à un diamètre ne dépassant pas 12 nm.

25. Composition non naturelle selon la revendication 1, dans laquelle chaque complexe consiste essentiellement en une molécule d'ADNc double brin unique et une ou plusieurs molécules de polycation.

26. Composition non naturelle selon la revendication 1, dans laquelle ladite molécule d'acide nucléique code au moins un acide nucléique anti-sens.

27. Composition non naturelle selon la revendication 1, dans laquelle ladite molécule d'acide nucléique est une molécule d'ARN.

28. Procédé de préparation d'une composition selon la revendication 10, dans lequel ledit mélange est effectué en l'absence de sel ajouté.

29. Composition selon les revendications 25, 26 ou 27 ou procédé selon la revendication 28, dans lesquels les molécules de polycation sont la polylysine ou un dérivé de polylysine.

30. Composition selon les revendications 25, 26 ou 27 ou procédé selon la revendication 28, dans lesquels le dérivé de polylysine est un peptide de polylysine avec un résidu de cystéine.

31. Complexes compactés solubles, non naturels d'un acide nucléique et d'une molécule de polycation, réalisés par le procédé selon l'une quelconque des revendications 9, 10 et 28.

32. Complexes selon la revendication 31, dans lesquels les molécules de polycation sont la polylysine ou un dérivé de polylysine.

33. Complexes selon la revendication 32, dans lesquels le dérivé de polylysine est un peptide de polylysine avec un résidu de cystéine.

34. Utilisation de la composition selon la revendication 1, dans la fabrication d'un médicament pour prévenir ou traiter une maladie ou une autre condition clinique chez un sujet, sur lequel le médicament est destiné à une administration intramusculaire ou dans le poumon du sujet,
ledit acide nucléique étant un acide dont l'intégration, l'hybridation ou l'expression dans les cellules cibles dudit sujet prévient ou traite ladite maladie ou toute autre condition clinique.

35. Utilisation selon la revendication 34, dans laquelle la maladie ou une autre condition clinique est choisie parmi les maladies pulmonaires, les troubles métaboliques et les cancers.

36. Utilisation selon la revendication 34, dans laquelle la maladie ou une autre condition clinique est la fibrose cystique.

37. Utilisation selon la revendication 34, dans laquelle le médicament est destiné à une administration par inhalation.

38. Utilisation selon la revendication 34, dans laquelle le médicament est destiné à l'administration par injection intramusculaire.

39. Utilisation selon la revendication 34, dans laquelle les molécules de polycation sont la polylysine ou un dérivé de polylysine.

40. Utilisation selon la revendication 39, dans laquelle le dérivé de polylysine est un peptide de polylysine avec un résidu de cystéine.

41. Composition selon la revendication 1, dans laquelle les complexes d'acide nucléique sont associés à un lipide.

42. Composition selon l'une quelconque des revendications 25, 26 et 27, dans laquelle le complexe d'acide nucléique est compacté à un diamètre ne dépassant pas 12 mm.

43. Procédé selon la revendication 28, dans lequel le mélange est contrôlé pour détecter, empêcher ou corriger la formation des complexes agglomérés ou relâchés.

44. Procédé selon la revendication 28, dans lequel le rapport molaire des groupes phosphate de l'acide nucléique aux groupes positivement chargés du polycation est dans la gamme de 4:1 à 1 :4.

45. Procédé selon la revendication 28, dans lequel l'acide nucléique est ajouté au polycation, tout en étant agité par vortex à grande vitesse.

46. Procédé selon la revendication 28, dans lequel le mélange est contrôlé par un procédé choisi dans le groupe constitué de microscopie électronique, diffusion de lumière, dichroïsme circulaire, et mesure d'absorption.

47. Composition selon la revendication 1, dans laquelle ledit polycation est un CK15-60P10 et le contre-ion est de l'acétate, dans laquelle le CK15-60P10 est un polymère polyacide aminé d'une cystéine N-terminal et 15-60 résidus lysine, dans laquelle une molécule de polyéthylène glycol ayant une masse moléculaire moyenne de 10 kDa est fixée au résidu cystéine.

48. Composition selon la revendication 19, dans laquelle le polycation est le CK15-60P10, et le contre-ion est l'acétate, dans laquelle le CK15-60 est un polymère polyacide aminé d'une cystéine N-terminal et 15-60 résidus lysine, dans laquelle une molécule de polyéthylène glycol ayant une masse moléculaire moyenne de 10 kDa est fixée au résidu cystéine.

49. Composition selon la revendication 25, dans laquelle ledit polycation est un CK15-60P10 et ledit contre-ion est de l'acétate, dans lequel le CK15-60P10 est un polymère polyacide aminé d'une cystéine N- terminal et 15-60 résidus lysine, dans laquelle une molécule de polyéthylène glycol ayant une masse moléculaire moyenne de 10 kDa est fixée au résidu cystéine.

50. Composition selon la revendication 26, dans laquelle ledit polycation est un CK15-60P10, et le contre-ion est de l'acétate, dans lequel le CK15-60P10 est un polymère polyacide aminé d'une cystéine N-terminal et 15-60 résidus lysine, dans laquelle une molécule de polyéthylène glycol ayant une masse moléculaire moyenne de 10 kDa est fixée au résidu cystéine.

51. Composition selon la revendication 27, dans laquelle ledit polycation est un CK15-60P10, et ledit contre-ion est de l'acétate, dans laquelle le CK15-60P10 est un polymère polyacide aminé d'une cystéine N-terminal et 15-60 résidus lysine, dans laquelle une molécule de polyéthylène glycol ayant une masse moléculaire moyenne de 10 kDa est fixée au résidu cystéine.

52. Procédé selon la revendication 28, dans lequel ledit polycation est un CK15-60P10, et ledit contre-ion est de l'acétate, dans laquelle le CK15-60P10 est un polymère polyacide aminé d'une cystéine N-terminal N et 15-60 résidus lysine, dans laquelle une molécule de polyéthylène glycol ayant une masse moléculaire moyenne de 10 kDa est fixée au résidu cystéine.

53. Composition selon l'une quelconque des revendications 47 à 51, dans laquelle la molécule de polycation comprend 30 résidus lysine.

54. Composition selon l'une quelconque des revendications 47 à 51, dans laquelle la molécule de polycation comprend une portion de ciblage.

55. Composition selon les revendications 47 ou 48, dans laquelle le complexe d'acide nucléique est compacté à un diamètre ne dépassant pas 12 nm.

56. Composition selon l'une quelconque des revendications 47 à 51, qui est lyophilisée.

57. Composition selon la revendication 47, qui est réhydratée après lyophilisation.

58. Composition selon l'une quelconque des revendications 47 à 51, qui ne contient pas de disaccharide.

59. Procédé *in vitro* de distribution de polynucléotides aux cellules comprenant :
la mise en contact de la composition de la revendication 57 avec des cellules, moyennant quoi l'acide nucléique est distribué aux cellules et prélevé par celles-ci.

60. Procédé selon la revendication 59, dans lequel la composition ne contient pas de disaccharide.

61. Composition non naturelle selon la revendication 48, dans laquelle ladite molécule d'acide nucléique comprend un promoteur qui contrôle la transcription d'une molécule d'ARN codant la protéine fonctionnelle.

62. Composition non naturelle selon la revendication 48, dans laquelle la protéine est thérapeutique.

63. Composition selon la revendication 48 qui est réhydratée après lyophilisation.

64. Procédé *in vitro* de distribution de polynucléotides aux cellules comprenant :
la mise en contact de la composition de la revendication 63 avec des cellules, moyennant quoi la protéine est exprimée.

65. Composition selon la revendication 49, qui est réhydratée après lyophilisation.

66. Procédé *in vitro* de distribution de polynucléotides aux cellules comprenant :
la mise en contact de la composition de la revendication 65 avec des cellules, dans lequel le polynucléotide code une protéine et moyennant quoi la protéine est exprimée.

67. Composition selon la revendication 50 qui est réhydratée après lyophilisation.

68. Composition selon la revendication 67, qui ne contient pas de disaccharide.

69. Composition selon la revendication 51 qui est lyophilisée et réhydratée.

70. Procédé *in vitro* de distribution de polynucléotides aux cellules comprenant :
la mise en contact de la composition de la revendication 69 avec des cellules, moyennant quoi le polynucléotide est distribué aux cellules et prélevé par celles-ci.

71. Procédé selon la revendication 52 comprenant en outre la lyophilisation des complexes d'acide nucléique non agglomérés.

72. Procédé selon la revendication 71, comprenant en outre la réhydratation des complexes d'acide nucléique lyophilisés.
